Europäisches Patentamt
European Patent Office
Office européen des brevets

(19) (11) Veröffentlichungsnummer: **0 069 838**
**A1**

(12) # EUROPÄISCHE PATENTANMELDUNG

(21) Anmeldenummer: **82103892.4**

(22) Anmeldetag: **05.05.82**

(51) Int. Cl.³: **C 07 C 103/26**
**A 61 K 31/165**

(30) Priorität: **08.05.81 CH 2998/81**

(43) Veröffentlichungstag der Anmeldung:
**19.01.83 Patentblatt 83/3**

(84) Benannte Vertragsstaaten:
**AT BE CH DE FR IT LI LU NL SE**

(71) Anmelder: **CIBA-GEIGY AG**
**Patentabteilung Postfach**
**CH-4002 Basel(CH)**

(72) Erfinder: **Ostermayer, Franz, Dr.**
**Am Hang 5**
**CH-4125 Riehen(CH)**

(72) Erfinder: **Zimmermann, Markus, Dr.**
**Steinbrecheweg 8**
**CH-4125 Riehen(CH)**

(72) Erfinder: **Fuhrer, Walter, Dr.**
**Munzackerweg 11**
**CH-4402 Frenkendorf(CH)**

(74) Vertreter: **Zumstein, Fritz sen., Dr. et al,**
**Bräuhausstrasse 4**
**D-8000 München 2(DE)**

(54) **Aralkylaminoethoxysalicylamide und deren pharmazeutische Verwendung.**

(57) Neue substituierte 2-Aminoäthanole der Formel

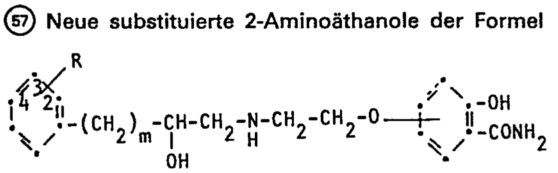

(1),

worin R Methyl, Methoxy, Fluor, Chlor, oder Brom bedeutet, und m für Null oder 3 steht, mit der Massgabe, dass, wenn m Null ist, R für 2-Methyl, 3-Methyl, 4-Methyl, 4-Fluor, 4-Chlor, 4-Brom, oder 4-Methoxy steht, und die basische Seitenkette über ihr Sauerstoffatom an den Salicylamidrest in 4-Stellung zur darin vorhandenen Hydroxygruppe gebunden ist, oder, wenn m Null ist und R für 4-Methyl steht, die basische Seitenkette über ihr Sauerstoffatom an den Salicylamidrest in 4-Stellung zur darin vorhandenen Carboxamidgruppe gebunden ist, oder, wenn m 3 bedeutet, R für 2-Methoxy steht und die basische Seitenkette über ihr Sauerstoffatom an den Salicylamidrest in 4-Stellung zur darin vorhandenen Hydroxygruppe gebunden ist, als Racemate, optische Antipoden oder deren Salze, können zur Behandlung von reaktiven oder endogenen Depressionen unterschiedlichen Schweregrades, sowie neurotischen oder anderen psychischen Störungen mit Antriebsschwäche und depressiven Verstimmungen verwendet werden. Ausserdem wirken solche Verbindungen teils als Blocker, teils als Stimulatoren β-adrenerger Rezeptoren mit mehr oder weniger ausgeprägter Cardioselektivität und können demnach einesteils bei den für β-Blocker üblichen Indikationen, anderenteils zur Behandlung der insuffizienten Herzleistung, von Asthma und Durchblutungsstörungen eingesetzt werden.

EP 0 069 838 A1

0069838



CIBA-GEIGY AG 4-13390/12619/+

Basel (Schweiz)

BEZEICHNUNG GEÄNDERT
siehe Titelseite

## Substituierte 2-Amino-äthanole

Die Erfindung betrifft neue substituierte 2-Aminoäthanole, Verfahren zu ihrer Herstellung und pharmazeutische Präparate enthaltend solche Verbindungen, und ihre Verwendung zur Herstellung von pharmazeutischen Präparaten, oder als pharmakologisch wirksame Verbindungen.

Die erfindungsgemässen neuen Verbindungen entsprechen der Formel

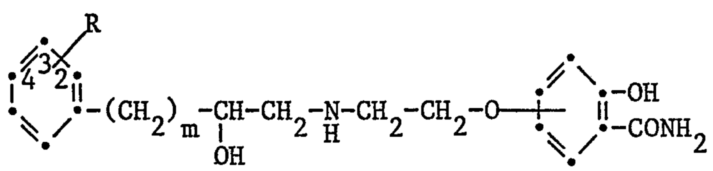

(I),

worin R Methyl, Methoxy, Fluor, Chlor oder Brom bedeutet, und m für Null oder 3 steht, mit der Massgabe, dass, wenn m Null ist, R für 2-Methyl, 3-Methyl, 4-Methyl, 4-Fluor, 4-Chlor, 4-Brom oder 4-Methoxy steht, und die basische Seitenkette über ihr Sauerstoffatom an den Salicylamidrest in 4-Stellung zur darin vorhandenen Hydroxygruppe gebunden ist, oder, wenn m Null ist und R für 4-Methyl steht, die basische Seitenkette über ihr Sauerstoffatom an den Salicylamidrest in 4-Stellung zur darin vorhandenen Carboxamidgruppe gebunden ist, oder, wenn m 3 bedeutet, R für 2-Methoxy steht und die basische Seitenkette über ihr Sauerstoffatom an den Salicylamidrest in 4-Stellung zur darin vorhandenen Hydroxygruppe gebunden ist, in der Form von Racematen, optischen Antipoden oder deren Salzen, insbesondere Säureadditionssalzen und vor allem pharmazeutisch annehmbaren, nicht-toxischen Säureadditionssalzen.

Die neuen Verbindungen besitzen wertvolle pharmakologische Eigenschaften. So zeigen sie zentralnervöse Wirkungen, die sich beispielsweise in der Aufhebung der Symptome der herabgesetzten sympathischen Funktionen und Aufhebung der Antriebsschwäche wiederspiegeln, wie sich z.B. aufgrund des Antagonismus gegenüber der an Mäusen nach s.c.-Verabreichung von 2 mg/kg Reserpin hervorgerufenen Hypothermie (B. Benz, P.G. Waser, Arzneimittelforschung 21, 654-61 (1971)) in einem Dosenbereich von etwa 3 mg/kg bis etwa 100 mg/kg i.p., oder aufgrund des Antagonismus gegenüber der an Mäusen nach s.c.-Verabreichung von 10 mg/kg Apomorphin hervorgerufenen Hypothermie (P.L. Puech; Europ. J. Pharmacol. 47, 125-127 (1978); E.L. Schelkunov: Psychopharmacol. 55, 87-95 (1977)) in einem Dosenbereich von etwa 0,3 mg/kg bis etwa 10 mg/kg i.p., oder aufgrund des Antagonismus gegenüber der an Mäusen nach i.p.-Verabreichung von 0,25 mg/kg Clonidin hervorgerufenen Hypothermie (P.F. Voigtländer et al: Neuropharmacol. 17, 375-381, (1978)) in einem Dosenbereich von etwa 0,05 mg/kg bis etwa 2,5 mg/kg nachweisen lässt.

Aufgrund dieser Wirksamkeiten erscheinen die genannten Präparate geeignet zur Behandlung von reaktiven oder endogenen Depressionen unterschiedlichen Schweregrades, sowie neurotischen oder anderen psychischen Störungen mit Antriebsschwäche und depressiven Verstimmungen. Die Verbindungen können auch für die Kurzbehandlung von postpartalen oder postoperativen Depressionen, oder von Depressionen anderen Ursprungs, allein oder in der Kombination mit anderen antidepressiv wirkenden Substanzen verwendet werden.

Die neuen Verbindungen der Formel I wirken ausserdem in spezifischer Weise auf β-adrenerge Rezeptoren. Dieser Wirkung liegt als gemeinsame Eigenschaft der Verbindungen der Formel I die Affinität zu diesen Rezeptoren zugrunde, die sich bei fehlender oder sehr geringer stimulierender Eigenwirkung als reine Blockade, bei geringer bis mittelstarker stimulierender Eigenwirkung als Blockade mit gleichzeitiger ISA, d.h. intrinsic sympathomimetic activity, und bei stärkerer Eigenwirkung als überwiegende Stimulierung der β-adrenergen Rezeptoren äussert. Die Grenzen zwischen β-Rezeptoren-Blockern ohne oder mit höchstens mittelstarker ISA sind fliessend, ebenso die therapeutischen Anwendungsbereiche dieser Verbindungstypen.

Die vorstehenden Angaben beruhen auf den Resultaten von entsprechenden pharmakologischen Versuchen in üblichen Testverfahren. So zeigen die neuen Verbindungen einerseits eine blockierende Wirkung an cardialen β-Rezeptoren, was anhand der Hemmung der durch Isoproterenol induzierten Tachykardie an isolierten Meerschweinchenherzen nach Langendorff in einem Konzentrationsbereich von etwa 0,01 µMol/Liter bis etwa 10 µMol/Liter nachweisbar ist, während eine blockierende Wirksamkeit an vaskulären β-Rezeptoren sich mittels der Hemmung der durch Isoproterenol induzierten Blutdrucksenkung an der narkotisierten Katze in einem Dosenbereich von etwa 0,1 mg/kg bis etwa 10 mg/kg nach intravenöser Verabreichung zeigen lässt. Die neuen Verbindungen bewirken ausserdem in einem Dosenbereich von etwa 0,1 mg/kg bis etwa 10 mg/kg i.v. eine Senkung des arteriellen Blutdruckes an der narkotisierten Katze. Aufgrund dieser Eigenschaften erscheinen die neuen Verbindungen als gegebenenfalls cardioselektive β-Rezeptorenblocker z.B. zur Behandlung von Angina pectoris und Herzrythmusstörungen sowie zur Verwendung als blutdrucksenkende Mittel geeignet.

Andererseits besitzen die neuen Verbindungen, insbesondere solche, in denen R eine Methylgruppe oder ein Fluoratom darstellt, und der Salicylamidrest in p-Stellung zur aromatischen Hydroxygruppe an das Sauerstoffatom der basischen Seitenkette gebunden ist, stimulierende Wirkungen an cardialen β-Rezeptoren, was sich anhand der positiv chronotropen Wirkung am spontan schlagenden, rechten isolierten Meerschweinchenvorhof in einem Konzentrationsbereich von etwa 0,01 µMol/Liter bis etwa 10 µMol/Liter nachweisen lässt.

Aufgrunddessen erscheinen solche Verbindungen als gegebenenfalls carioselektive β-Rezeptorenstimulatoren einerseits zur Behandlung der insuffizienten Herzleistung, andererseits als Broncho- und Vasodilatoren zur Behandlung von Asthma und Durchblutungsstörungen geeignet.

Die neuen Verbindungen der Formel I werden in an sich bekannter Weise hergestellt. Man kann sie z.B. erhalten, indem man eine Verbindung der Formel

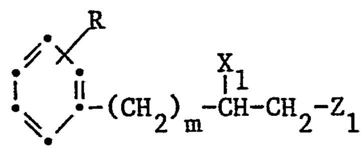

(II)

mit einer Verbindung der Formel

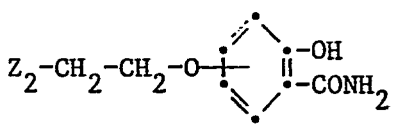

(III),

worin eine der Gruppen $Z_1$ und $Z_2$ eine reaktionsfähige veresterte Hydroxygruppe darstellt und die andere für die primäre Aminogruppe steht, und $X_1$ Hydroxy bedeutet, oder worin $X_1$ und $Z_1$ zusammen die Epoxygruppe bedeuten und $Z_2$ für die primäre Aminogruppe steht, R und m obige Bedeutung haben, umsetzt, und, wenn erwünscht, eine erhaltene freie Verbindung in ein Salz oder ein erhaltenes Salz in eine freie Verbindung überführt, und/oder, wenn erwünscht, ein erhaltenes Racemat in die optischen Antipoden auftrennt.

Eine reaktionsfähige veresterte Hydroxygruppe $Z_1$ bzw. $Z_2$ ist eine durch eine starke Säure, insbesondere eine starke anorganische Säure, wie eine Halogenwasserstoffsäure, insbesondere Chlor-, Brom- oder Jodwasserstoffsäure, oder Schwefelsäure, oder eine starke organische Säure, insbesondere eine starke organische Sulfonsäure, wie eine aliphatische oder aromatische Sulfonsäure, z.B. Methansulfonsäure, 4-Methylbenzolsulfonsäure oder 4-Brombenzolsulfonsäure, veresterte Hydroxygruppe, und stellt in erster Linie Halogen, z.B. Chlor, Brom oder Jod, oder aliphatisch oder aromatisch substituiertes Sulfonyloxy, z.B. Methylsulfonyloxy oder 4-Methylphenylsulfonyloxy dar.

Die obige Reaktion wird in an sich bekannter Weise durchgeführt, wobei man, besonders bei Verwendung eines Ausgangsmaterials mit einer reaktionsfähigen veresterten Hydroxygruppe, vorteilhafterweise in Gegenwart eines basischen Mittels, wie einer anorganischen Base, z.B. eines Alkalimetall- oder Erdalkalimetallcarbonats oder -hydroxids, oder eines organischen basischen Mittels, wie eines Alkalimetall-niederalkanolats, und/oder eines Ueberschusses des basischen Reaktionsteilnehmers und üblicherweise in Gegenwart, aber gegebenenfalls auch in Abwesenheit eines Lösungsmittels oder Lösungsmittelgemisches und, wenn notwendig, unter Kühlen oder Erwärmen, z.B. in einem Temperaturbereich von etwa -20°C bis etwa +150°C, in einem offenen oder geschlossenen Gefäss und/oder in einer Inertgasatmosphäre, z.B. in einer Stickstoffatmosphäre, arbeitet.

Ausgangsstoffe der Formeln II oder III sind bekannt oder können in an sich bekannter Weise hergestellt werden. So kann man zur Herstellung eines Ausgangsstoffes der Formel II, worin m für 0 steht, eine Verbindung der Formel

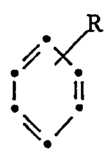

(IIa)

mit einem Halogenacetylhalogenid, z.B. Chloracetylchlorid, in Gegenwart einer geeigneten Lewissäure, z.B. Aluminiumchlorid, nach der Friedel-Crafts-Methode an einem Kohlenstoffatom des aromatischen Restes halogenacetylieren und in der so erhältlichen entsprechenden Halogenacetyl-Verbindung, z.B. durch Behandeln mit einem geeigneten Hydridreduktionsmittel, die Carbonyl- zur Carbinolgruppe reduzieren; wenn erwünscht, kann man ein Halogen $Z_1$ z.B. durch Behandeln mit Ammoniak, oder einem geeigneten Derivat davon, wie Hexamethylentetramin, und Zersetzung der erhaltenen Verbindung mit verdünnter Mineralsäure, oder durch Umsetzen mit einem Alkalimetallsalz des Phthalimids und Spalten der erhaltenen N-Phthalimid-Verbindung, z.B. mit Hydrazin, in die primäre Aminogruppe $Z_1$ umwandeln. Ausgangsstoffe der Formel II, worin $X_1$ und $Z_1$ zusammen Epoxy bedeuten, können z.B.

durch Cyclisierung einer Verbindung der Formel II, worin $X_1$ Hydroxy und $Z_1$ eine reaktionsfähige veresterte Hydroxygruppe, etwa Chlor oder Methansulfonyloxy darstellen, mittels alkalischer Reagentien, z.B. eines Gemisches von verdünnter Natronlauge und Tetrabutylammoniumchlorid in einem geeigneten Lösungsmittel, z.B. Methylenchlorid, erhalten werden.

Ausgangsstoffe der Formel II, worin m obige Bedeutung hat und $X_1$ und $Z_1$ zusammen die Epoxygruppe bedeuten, können erhalten werden, indem man in einer Verbindung der Formel

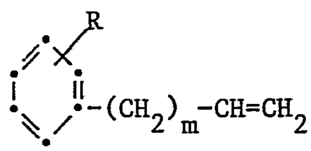

(IIb)

die Gruppe $-CH=CH_2$ durch Epoxidieren in die Gruppe

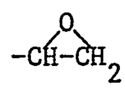

umwandelt, z.B. durch Umsetzen mit einer Peroxy-Verbindung, wie Wasserstoffperoxid, oder einer organischen Persäure, wie z.B. einer gegebenenfalls substituierten, wie halogenierten, aliphatischen oder aromatischen Persäure, z.B. Peressigsäure oder Trifluorperessigsäure, Perbenzoesäure oder m-Chlorperbenzoesäure, in einem wasserfreien Lösungsmittel, etwa einem Halogen-niederalkan, wie Chloroform oder Methylenchlorid. Diese Umsetzung wird in üblicher Weise vorgenommen.

Ausgangsstoffe der Formel IIb wiederum, worin m für 3 steht, können durch Umsetzung einer Verbindung der Formel

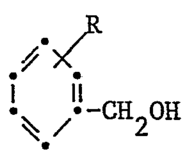

(IIc),

worin die Hydroxygruppe reaktionsfähig verestert, z.B. wie oben angegeben, ist, und z.B. für Halogen, etwa Brom, steht, mit einer Grignard-Verbindung, z.B. mit einer Verbindung der Formel

$Br-(CH_2)_n-CH=CH_2$ (IId), worin n für 1 oder 2 steht, in üblicher Weise erhalten werden.

Ausgangsstoffe der Formel III können z.B. durch Umsetzung eines Hydroxysalicylamids mit einem 1,2-Dihalogenäthan, etwa 1-Chlor-2-brom- oder 1,2-Dibromäthan, in Gegenwart eines alkalischen Kondensationsmittels, wie einem Alkalicarbonat, und, wenn erwünscht, Ersatz des verbleibenden Halogens $Z_2$ in der oben für $Z_1$ angegebenen Weise durch die primäre Aminogruppe, erhalten werden. Diese Umsetzungen werden in üblicher Weise vorgenommen.

Die Verbindungen der Formel I können weiterhin hergestellt werden, indem man in einer Verbindung der Formel

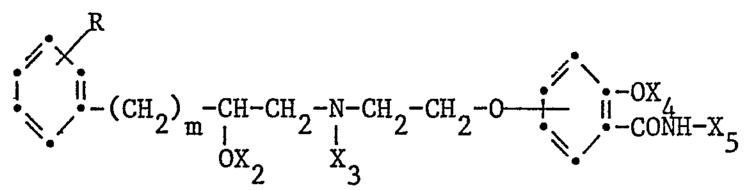 (IV),

worin $X_2$, $X_3$, $X_4$ und $X_5$ jeweils Wasserstoff oder einen durch Wasserstoff ersetzbaren Substituenten bedeuten, oder $X_2$ und $X_3$ und/oder $X_4$ und $X_5$ zusammen einen zweiwertigen, durch zwei Wasserstoffatome ersetzbaren Rest bedeuten mit der Massgabe, dass mindestens einer der Reste $X_2$, $X_3$, $X_4$ und $X_5$ von Wasserstoff verschieden ist, oder mindestens $X_2$ und $X_3$ zusammen oder $X_4$ und $X_5$ zusammen einen zweiwertigen, durch zwei Wasserstoffatome ersetzbaren Rest darstellen, und R und m obige Bedeutungen haben, oder in einem Salz davon, das von Wasserstoff verschiedene $X_2$, $X_3$, $X_4$ oder $X_5$ bzw. $X_2$ und $X_3$ zusammen oder $X_4$ und $X_5$ zusammen durch Wasserstoff ersetzt, und wenn erwünscht, die anschliessend an das erste Verfahren genannten zusätzlichen Verfahrensschritte durchführt.

Die Abspaltung der Gruppen $X_2$,$X_3$,$X_4$ oder $X_5$ oder jeweils $X_2$ und $X_3$ oder $X_4$ und $X_5$ zusammen wird mittels Solvolyse, wie Hydrolyse, Alkoholyse, Aminolyse oder Acidolyse, oder mittels Reduktion einschliesslich

Hydrogenolyse vorgenommen.

Besonders geeignete, abspaltbare Gruppen $X_3$ und $X_4$ sind in erster Linie hydrogenolytisch abspaltbare α-Arylniederalkylgruppen, wie eine gegebenenfalls substituierte 1-Polyphenyl- oder 1-Phenylniederalkylgruppe, worin Substituenten, insbesondere des Phenylteils, z.B. Niederalkyl, wie Methyl, oder Niederalkoxy, wie Methoxy sein können, und in erster Linie Benzyl. Gruppen $X_3$ und insbesondere $X_2$ und $X_4$ können auch solvolytisch, wie hydrolytisch oder acidolytisch, ferner reduktiv, einschliesslich hydrogenolytisch abspaltbare Reste, insbesondere entsprechende Acylreste,wie den Acylrest einer organischen Carbonsäure, z.B. Niederalkanoyl, wie Acetyl, oder Aroyl, wie Benzoyl, ferner den Acylrest eines Halbesters der Kohlensäure, wie Niederalkoxycarbonyl, z.B. Methoxycarbonyl, Aethoxycarbonyl oder tert.-Butyloxycarbonyl, 2-Halogenniederalkoxycarbonyl, z.B. 2,2,2-Trichloräthoxycarbonyl oder 2-Jodäthoxycarbonyl, gegebenenfalls substituiertes 1-Phenylniederalkoxycarbonyl, z.B. Benzyloxycarbonyl oder Diphenylmethoxycarbonyl, oder Aroylmethoxycarbonyl, z.B. Phenacyloxycarbonyl, ferner gegebenenfalls substituierte 1-Polyphenylniederalkylgruppen, worin Substituenten, in erster Linie des Phenylteils, z.B. die oben gegebene Bedeutung haben, und in erster Linie Trityl darstellen.

Ein durch $X_2$ und $X_3$ und/oder $X_4$ und $X_5$ zusammen gebildeter, abspaltbarer Rest ist in erster Linie eine hydrogenolytisch abspaltbare zweiwertige Gruppe, wie gegebenenfalls substituiertes 1-Phenylniederalkyliden, worin Substituenten, insbesondere des Phenylteils, z.B. Niederalkyl oder Niederalkoxy sein können, und insbesondere Benzyliden, sowie solvolytisch, insbesondere hydrolytisch, abspaltbare Gruppen, wie Niederalkyliden, z.B. Methylen oder Isopropyliden, oder 1-Phenyl-niederalkyliden, dessen Phenylteil gegebenenfalls durch Niederalkyl, wie Methyl, oder Niederalkoxy, wie Methoxy, substituiert ist, insbesondere Benzyliden, oder Cycloalkyliden, z.B. Cyclopentyliden oder Cyclohexyliden.

In der Form von Salzen verwendbare Ausgangsstoffe werden in erster Linie in der Form von Säureadditionssalzen, z.B. mit Mineralsäuren, sowie mit organischen Säuren verwendet.

Hydrogenolytisch abspaltbare Reste $X_2$, $X_3$ und/oder $X_4$, insbesondere gegebenenfalls substituierte 1-Phenylniederalkylgruppen, ferner auch geeignete Acylgruppen, wie gegebenenfalls substituiertes 1-Phenylniederalkoxycarbonyl, sowie durch die Gruppen $X_2$ und $X_3$ sowie $X_4$ und $X_5$ zusammen gebildete, gegebenenfalls substituierte 1-Phenylniederalkylidengruppen, können durch Behandeln mit katalytisch aktiviertem Wasserstoff, z.B. mit Wasserstoff in Gegenwart eines Nickelkatalysators, wie Raney-Nickel, oder eines geeigneten Edelmetallkatalysators, abgespalten werden.

Hydrolytisch abspaltbare Gruppen $X_2$, $X_3$ und/oder $X_4$ wie Acylreste von organischen Carbonsäuren, z.B. Niederalkanoyl, und von Halbestern der Kohlensäure, z.B. Niederalkoxycarbonyl, ferner z.B. Tritylreste, sowie durch die Reste $X_2$ und $X_3$ und/oder $X_4$ und $X_5$ zusammen gebildete Niederalkyliden-, 1-Phenyl-niederalkyliden- oder Cycloalkylidengruppen können je nach Art solcher Reste durch Behandeln mit Wasser unter sauren oder basischen Bedingungen, z.B. in Gegenwart einer Mineralsäure, wie Chlorwasserstoff- oder Schwefelsäure, oder eines Alkalimetall- oder Erdalkalimetallhydroxids oder -carbonats oder eines Amins wie Isopropylamin, abgespalten werden.

Durch Aminolyse können beispielweise als Schutzgruppen von Hydroxygruppen vorliegende Acylreste und insbesondere durch $X_4$ und $X_5$ zusammen gebildete Niederalkyliden-, 1-Phenylniederalkyliden- oder Cycloalkylidengruppen, wie z.B. die 1-Methyläthylidengruppe, abgespalten werden,

beispielsweise durch Umsetzen mit Ammoniak oder, besonders im Falle der vorgenannten zweiwertigen Schutzgruppen, mit primären Aminen, wie z.B. Isopropylamin oder Benzylamin, in einem geeigneten Reaktionsmedium, wie z.B. Isopropanol oder Dioxan.

Acidolytisch abspaltbare Reste $X_2$, $X_3$ und/oder $X_4$ sind insbesondere gewisse Acylreste von Halbestern der Kohlensäure, wie z.B. tert.-Niederalkoxycarbonyl oder gegebenenfalls substituierte Diphenylmethoxycarbonylreste, ferner auch der tert.-Butylrest; solche Reste können durch Behandeln mit geeigneten starken organischen Carbonsäuren, wie gegebenenfalls durch Halogen, insbesondere Fluor, substituierte Niederalkancarbonsäuren, in erster Linie mit Trifluoressigsäure (wenn notwendig, in Gegenwart eines aktivierenden Mittels, wie Anisol), sowie mit Ameisensäure abgespalten werden.

Unter reduktiv abspaltbaren Resten $X_2$, $X_3$ und/oder $X_4$ werden auch solche Gruppen verstanden, die beim Behandeln mit einem chemischen Reduktionsmittel (insbesondere mit einem reduzierenden Metall oder einer reduzierenden Metallverbindung) abgespalten werden. Solche Reste sind insbesondere 2-Halogenniederalkoxycarbonyl oder Arylmethoxycarbonyl, die z.B. beim Behandeln mit einem reduzierenden Schwermetall, wie Zink, oder mit einem reduzierenden Schwermetallsalz, wie einem Chrom(II)salz, z.B. -chlorid oder -acetat, üblicherweise in Gegenwart einer organischen Carbonsäure, wie Ameisensäure oder Essigsäure, und von Wasser abgespalten werden können.

Die obigen Reaktionen werden üblicherweise in Gegenwart eines Lösungsmittels, oder Lösungsmittelgemisches durchgeführt, wobei geeignete Reaktionsteilnehmer gleichzeitig auch als solche funktionieren können,

und, wenn notwendig, unter Kühlen oder Erwärmen, z.B. in einem offenen oder geschlossenen Gefäss und/oder in der Atmosphäre eines Inertgases, z.B. Stickstoff.

Die Ausgangsstoffe der Formel IV lassen sich in an sich bekannter Weise erhalten, indem man zur Herstellung eines Ausgangsstoffes, worin m für 0 steht, z.B. eine Verbindung der Formel

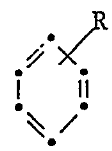

(IIa)

mit einem Halogenacetylhalogenid, z.B. Chloracetylchlorid, in Gegenwart einer geeigneten Lewissäure, z.B. Aluminiumchlorid, nach dem Friedel-Crafts-Verfahren umsetzt, die Carbonylgruppe in der so erhaltenen oder in anderer, üblicher Weise erhaltenen entsprechenden Halogenacetyl-Verbindung, z.B. mittels Natriumborhydrid, zur Carbinolgruppe reduziert und die erhaltene Verbindung mit einem Amin der Formel

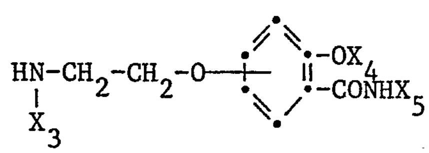

(IVa),

worin $X_3$ die angegebene Bedeutung hat, und $X_4$ oder $X_4$ und $X_5$ zusammen verschieden von Wasserstoff sind, umsetzt.

Die Ausgangsstoffe der Formel IV, worin m obige Bedeutung hat, sind durch Umsetzung einer Verbindung der oben erläuterten Formel II mit einer Verbindung der Formel IVa zugänglich. Diese Umsetzung wird in an sich bekannter Weise vorgenommen.

Man kann ferner z.B. die durch Umsetzung einer Verbindung der Formel

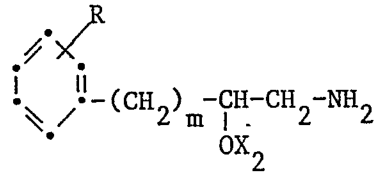

(IVb)

mit einer Verbindung der Formel

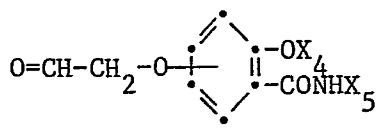

(IVc),

worin $X_4$ oder $X_4$ und $X_5$ zusammen eine der angegebenen Schutzgruppen bedeuten, gebildete Schiff'sche Base mit einem Borhydrid, etwa Natriumborhydrid, zur Verbindung der Formel IV reduzieren. Die Reduktion kann auch mittels aktiviertem Wasserstoff in Gegenwart eines Hydrierkatalysators, z.B. eines Platin-auf-Kohle Katalysators, erfolgen.

Amine der Formel IVb, worin $X_2$ Wasserstoff oder eine mittels Reduktion einschliesslich Hydrogenolyse abspaltbare und durch Wasserstoff ersetzbare Gruppe darstellt, können z.B. durch Umsetzung einer Verbindung der Formel

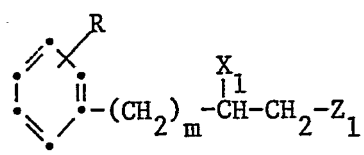

(IVd),

worin $X_1$ und $Z_1$ die unter der Formel (II) angegebene Bedeutung haben, mit Ammoniak oder einem einen mittels Reduktion einschliesslich Hydrogenolyse, z.B. wie angegeben, abspaltbaren und durch Wasserstoff ersetzbaren Rest enthaltenden Amin, z.B. Benzylamin, und nachfolgende Abspaltung dieses Restes, hergestellt werden.

Ausgangsstoffe der Formel IVd wiederum, worin $X_1$ und $Z_1$ zusammen die Epoxygruppe bedeuten, können analog, z.B. wie für die Herstellung von Verbindungen der Formel II beschrieben, etwa ausgehend von Verbindungen der Formel IIa über Verbindungen der Formel IIb, etwa wie beschrieben, und nachfolgender Epoxidierung, erhalten werden.

Carbonylverbindungen der Formel (IVc) können durch Umsetzung einer Verbindung der Formel

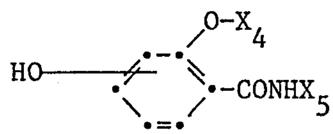 (IVe)

mit z.B. Chloracetaldehyd (IVf) in üblicher Weise erhalten werden.

Aus den vorgenannten Verbindungen der Formel (IVe) kann man auch durch Umsetzung mit z.B. 1,2-Dibromäthan eine Verbindung der Formel

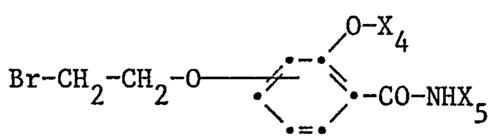 (IVg),

in der $X_4$ und $X_5$ die oben angegebene Bedeutung haben, herstellen und diese mit Verbindungen der Formel (IVb) zu Ausgangsstoffen der Formel IV umsetzen.

Die neuen Verbindungen der Formel I können ebenfalls erhalten werden, indem man in einer Verbindung der Formel

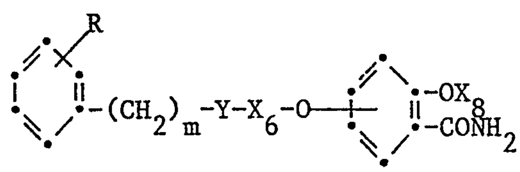 (V),

worin $X_6$ eine reduzierbare Gruppe der Formeln $-CH=N-CH_2-CH_2-$ (Va), bzw. $-CH_2-N=CH-CH_2-$ (Vb), oder $-C(=X_7)-N(X_8)-CH_2-CH_2-$ (Vc) bzw. $-CH_2-N(X_8)-X(=X_7)-CH_2-$ (Vd) oder eine Gruppe $-CH_2-N(X_8)-CH_2-CH_2-$ (Ve) ist, worin $X_7$ den Oxo- oder Thioxorest und $X_8$ Wasserstoff oder einen unter den Bedingungen für die Reduktion von $X_6$ und/oder Y durch Wasserstoff ersetzbaren Rest darstellt und Y für einen Rest der Formel -CO- (Vf) oder $-CH(OX_8)-$ (Vg) steht, in der $X_8$ die vorstehend angegebene Bedeutunghat, R und m obige Bedeutungen haben, wobei stets $X_6$ eine reduzierbare Gruppe Va bis Vd und/oder Y eine Carbonylgruppe Vf ist, diese Gruppe(n) reduziert und im gleichen Arbeitsgang die von Wasserstoff verschiedenen Gruppen $X_8$ durch Wasserstoff ersetzt, und, wenn erwünscht, die zusätzlichen, anschliessend an das erste Verfahren

genannten Verfahrensschritte durchführt.

Eine hydrogenolytisch abspaltbare Gruppe $X_8$ ist in erster Linie eine α-Arylniederalkylgruppe, wie eine gegebenenfalls substituierte 1-Phenylniederalkylgruppe, worin Substituenten z.B. Niederalkoxy, wie Methoxy, sein können, und ganz besonders Benzyl.

Ausgangsstoffe der Formel V mit einer Gruppe $X_6$ der Formel Vb können auch in der isomeren Form von Ring-Tautomeren der Formel

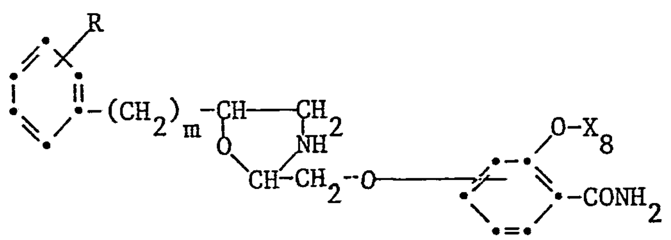

(Vh),

worin das Sauerstoff- und Stickstoffatom des Ringes an das gleiche Kohlenstoffatom gebunden sind, vorliegen.

Die Reduktion der Stickstoff-Kohlenstoff-Doppelbindung in Ausgangsstoffen der Formel V, die als $X_6$ eine Gruppe Va oder Vb enthalten, während Y und $X_8$ die unter der Formel V angegebene Bedeutung haben (oder in den isomeren Verbindungen der Formel Vh der Sauerstoff Kohlenstoff-Stickstoff-Bindung) zur Stickstoff-Kohlenstoff-Einfachbindung kann in an sich bekannter Weise, z.B. durch Behandeln mit katalytisch aktiviertem Wasserstoff, wie Wasserstoff in Gegenwart eines geeigneten Hydrierkatalysators, z.B. eines Nickel-, Platin- oder Palladiumkatalysators, erfolgen, wobei hydrogenolytisch abspaltbare Gruppen $X_8$ zugleich abgespalten und durch Wasserstoff ersetzt werden; oder man arbeitet mit einem geeigneten Hydridreduktionsmittel, wie einem Alkalimetallborhydrid, z.B. Natriumborhydrid. In allen Fällen wird zugleich mit der Gruppe Va oder Vb auch, falls vorhanden, ein Carbonylrest Y zum Hydroxymethylenrest reduziert, und bei Anwendung von Hydridreduktionsmitteln können auch an Sauerstoff gebundene Acylreste von Carbonsäuren, wie z.B. Essigsäure, als Reste $X_8$ vorliegen und im gleichen Arbeitsgang abgespalten werden.

Die Reduktion der Carbonylgruppe Y in Ausgangsstoffen der allgemeinen Formel V, die als Rest $X_6$ eine Gruppe Ve enthalten, während $X_8$ die unter der Formel V angegebene Bedeutung hat, kann in der vorstehend für die Reduktion der Gruppe Va und Vb angegebenen Weise erfolgen, wobei wiederum bei der katalytischen Hydrierung entsprechende Reste $X_8$ hydrogenolytisch abgespalten werden können.

Insbesondere geeignet zur Reduktion von Verbindungen der Formel V mit einer Gruppe der Formel Ve oder Vd und der unter der Formel V definierten Bedeutung von Y, sind Hydridreduktionsmittel, wie z.B. Natriumborhydrid oder Diboran.

Zugleich mit der Reduktion einer Gruppe Vc oder Vd erfolgt die Reduktion einer Carbonylgruppe Y, falls diese vorhanden ist, sowie die Abspaltung von an Sauerstoff gebundenen Acylresten von Carbonsäuren, wie z.B. Essigsäure, als Reste $X_8$. Andererseits ist durch Beschränkung der Menge Reduktionsmittel und geeignete Wahl der Reduktionsbedingungen dafür zu sorgen, dass die aromatisch gebundene Carboxamidgruppe nicht reduziert wird. Gruppierungen der Formeln Vc, Vd, worin $X_7$ jeweils eine Thioxogruppe bedeutet, werden durch reduktive Entschwefelung,z.B. durch Behandeln mit einem Hydrierkatalysator, wie Raney-Nickel, in die Gruppierung der Formel $-CH_2-NH-alk-$ umgewandelt. Die obigen Reduktionsreaktionen werden in an sich bekannter Weise, üblicherweise in Gegenwart eines inerten Lösungsmittels und, wenn notwendig, unter Kühlen oder Erwärmen, z.B. in einem Temperaturbereich von etwa -20° bis etwa +150°C, und/oder in einem geschlossenen Gefäss unter Druck und/ oder in einer Inertgas-, z.B. Stickstoffatmosphäre, durchgeführt.

Ein Ausgangsmaterial der Formel V, worin m für 0 steht, kann in an sich bekannter Weise, gegebenenfalls in situ, d.h. unter den Bedingungen des beschriebenen Verfahrens, hergestellt werden. So kann man eine Verbindung der Formel

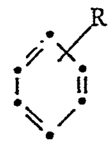

(Vi)

mit einem Essigsäurehalogenid oder -anhydrid in Gegenwart einer Lewissäure acetylieren und in dem erhaltenen Zwischenprodukt die Acetylgruppe dann z.B. durch Behandeln mit einem geeigneten Oxidationsmittel, wie Selendioxid, in die Glyoxyloylgruppe umwandeln. Eine solche Glyoxylverbindung, oder, wenn erwünscht, ein geeignetes Derivat derselben, etwa ein Acetal, kann dann mit einem Amin der Formel

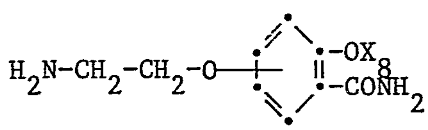

(Vj)

zu einem Ausgangsprodukt der Formel V mit der Gruppe $X_6$ der Formel Va umgesetzt werden, worin Y die Carbonylgruppe darstellt. Man kann ferner eine Verbindung der Formel (Vi) mit einem Halogenacetylhalogenid, z.B. Chloracetylchlorid, in Gegenwart einer geeigneten Lewissäure, z.B. Aluminiumchlorid, nach der Friedel-Crafts-Methode zur entsprechenden Chloracetyl-Verbindung halogenacetylieren, in der so oder in anderer üblicher Weise erhältlichen Halogenacetylverbindung durch Behandeln mit einem geeigneten Hydridreduktionsmittel die Carbonyl- zur Carbinolgruppe reduzieren, und das Halogenatom durch Behandeln mit Ammoniak oder einem geeigneten Derivat davon, wie Hexamethylentetramin, und Zersetzen des gebildeten Reaktionsproduktes mit verdünnter Säure, etwa wässriger Salzsäure, in die primäre Aminogruppe umwandeln, wonach man ein Zwischenprodukt der Formel

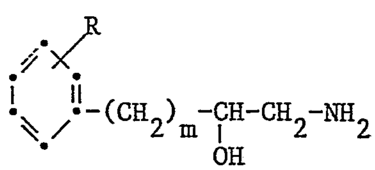

(Vk)

erhält, worin m für 0 steht. Amine der Formel Vk wiederum, worin m obige Bedeutung hat, sind z.B. durch Umsetzung einer Verbindung der Formel

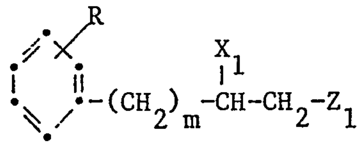

(Vl) ,

worin $X_1$ und $Z_1$ die unter der Formel (II) angegebene Bedeutung haben, mit Ammoniak oder einer Ammoniak liefernden Verbindung, z.B. Hexamethylentetramin, in bekannter Weise erhältlich, wobei Verbindungen der Formel (Vl) analog dem für die Herstellung von Ausgangsstoffen der Formel (II) beschriebenen Verfahren erhalten werden können.

Amine der Formel Vk, worin m obige Bedeutung hat, können ausserdem durch Umsetzung eines Aldehyds der Formel

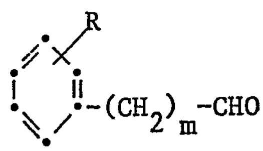

(Vm)

oder eines geeigneten Derivats davon, etwa eines Acetals, z.B. des Dimethylacetals, oder einer Bisulfitadditionsverbindung,mit einer geeigneten Silicium-organischen Cyanoverbindung, etwa einem Triniederalkyl-, z.B. Trimethylsilylcyanid, in üblicher Weise in Gegenwart eines Katalysators, wie Zinkjodid, zu einer Verbindung der Formel

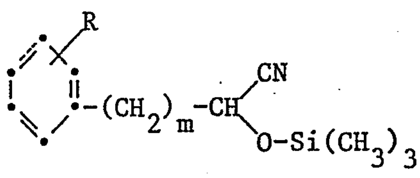

(Vn)

und deren Umwandlung mittels eines geeigneten Reduktionsmittels, z.B. eines Dileichtmetallhydrids, etwa Lithiumaluminiumhydrid, in einem Lösungsmittel, wozu üblicherweise eine ätherartige Flüssigkeit, z.B. Diäthyläther oder Tetrahydrofuran dient, in eine Verbindung der Formel Vk erhalten werden, worin m obige Bedeutung hat.

Durch Umsetzung eines Zwischenprodukts der Formel Vk mit einer Carbonylverbindung der Formel

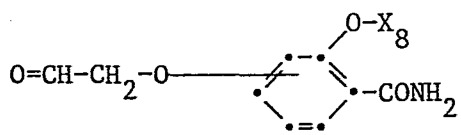
(Vo),

kann man zu Ausgangsstoffen der Formel V mit einer Gruppe $X_6$ der Formel (Vb) gelangen. Eine Modifizierung dieser Umsetzungen besteht darin, dass man in dem vorhin beschriebenen Zwischenprodukt das Halogenatom, statt es durch Behandeln mit Ammoniak etc. gegen die primäre Aminogruppe auszutauschen, durch Umsetzung mit einem 1-Aryl-niederalkylamin, z.B. Benzylamin oder einem Di-(1-aryl-niederalkyl)-amin z.B. Dibenzylamin gegen die entsprechende 1-Arylniederalkylamino- bzw. Di-(1-aryl-niederalkyl)-aminogruppe austauscht und die erhaltene Verbindung, etwa die entsprechende Dibenzylaminoverbindung, mit der Oxoverbindung der Formel Vo unter den reduzierenden Bedingungen des Verfahrens umsetzt. Hierbei verwendet man als Reduktionsmittel in erster Linie katalytisch aktivierten Wasserstoff, z.B. Wasserstoff in Gegenwart eines Schwermetallhydrierkatalysators oder eines Gemisches davon, wie eines Palladium- und/oder Platinkatalysators. Unter solchen Reaktionsbedingungen werden hydrogenolytisch abspaltbare Gruppen $X_8$, z.B. Benzylgruppen, abgespalten, die gegebenenfalls vorhandene Carbonyl- zur Carbinolgruppe und gleichzeitig die Stickstoff-Kohlenstoff-Doppelbindung zur entsprechenden Stickstoff-Kohlenstoff-Einfachbindung reduziert.

Oxoverbindungen der Formel Vo wiederum sind z.B. durch Umsetzung einer Dihydroxyverbindung der Formel

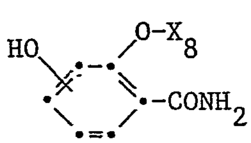
(Vp)

mit Chloracetaldehyd in Gegenwart eines alkalischen Kondensationsmittels, etwa Kaliumcarbonat, oder einer organischen Base, wie Triäthylamin, erhältlich.

Ausgangsprodukte der Formel V mit einer Gruppe $X_6$ der Formel Vc oder Vd können in an sich bekannter Weise hergestellt werden, indem man z.B. eine Formylverbindung der Formel

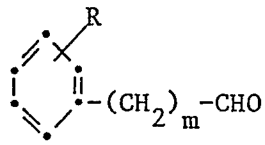

(Vq)

mit Cyanwasserstoff umsetzt und im so erhältlichen Cyanhydrin-Zwischenprodukt die Cyan- zur Carboxylgruppe, z.B. unter sauren Bedingungen, hydrolysiert. Die so oder über die Zwischenstufen Imidchlorid, Imidoniederalkylester und Niederalkylester erhaltene entsprechende 2-Hydroxyessigsäure wird dann in Gegenwart eines geeigneten Kondensationsmittels, z.B. eines Carbodiimids, wie Dicyclohexylcarbodiimid, mit einem Amin der Formel (Vj) umgesetzt, wonach man ein Ausgangsmaterial der Formel V mit der Gruppe $X_6$ der Formel (Vc) erhält.

Ferner kann man durch Umsetzung einer Verbindung der Formel Vk mit einer Verbindung der Formel

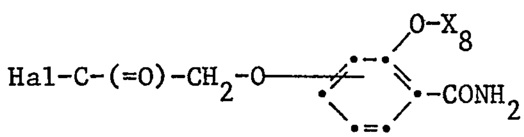

(Vr),

worin Hal für Halogen und insbesondere für Chlor steht, ein Ausgangsmaterial der Formel (V) mit der Gruppe $X_6$ der Formel (Vd) erhalten. Man kann ferner in der oben angegebenen Chloracetylverbindung das Chlor etwa durch Umsetzung mit Hexamethylentetramin gegen die primäre Aminogruppe austauschen und eine so erhaltene Verbindung mit einer Halogenverbindung der Formel Vr umsetzen. In einem so erhaltenen Ausgangsprodukt der Formel V mit einer Gruppe $X_6$ der Formel Vd und worin Y die Carbonylgruppe darstellt, kann man die Carbonyl- zur Carbinolgruppe und die aliphatisch gebundene Carbamoylgruppe zur Gruppe der Formel $-CH_2-\underset{H}{N}-alk-$ gleichzeitig reduzieren, etwa mittels eines Hydridreduktionsmittels, insbesondere Diboran.

Die neuen Verbindungen der Formel I können ebenfalls erhalten werden,

indem man eine Verbindung der Formel

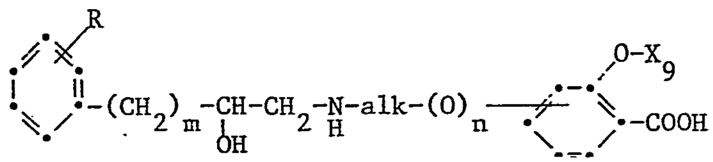

(VI),

worin $X_9$ Wasserstoff oder eine mittels Ammonolyse abspaltbare Gruppe bedeutet, R und m obige Bedeutungen haben, oder ein reaktionsfähiges Derivat einer der in Formel VI definierten Carbonsäuren, mit Ammoniak (VII) umsetzt und gegebenenfalls vorhandene Reste $X_9$ abspaltet und durch Wasserstoff ersetzt, und, wenn erwünscht, die anschliessend an das erste Verfahren genannten zusätzlichen Verfahrensschritte durchführt. Mittels Ammonolyse abspaltbare Reste $X_9$ sind Acylreste von organischen Carbonsäuren, z.B. Aroyl, wie Benzoyl, oder Niederalkanoyl, wie Acetyl.

Reaktionsfähige Derivate der in Formel VI definierten Carbonsäuren sind z.B. die Halogenide, wie die Chloride oder Bromide, ferner die Azide, sowie Säureanhydride, insbesondere gemischte Säureanhydride mit z.B. Niederalkancarbonsäuren, wie Essigsäure oder Propionsäure, Niederalkoxyalkancarbonsäuren, wie 2-Methoxyessigsäure. Reaktionsfähige Derivate von Carbonsäuren der Formel VI sind insbesondere Ester, z.B. mit Niederalkanolen, wie Methanol, Aethanol, Isopropanol, tert.-Butanol, ferner mit Arylniederalkanolen, etwa gegebenenfalls durch Niederalkyl, z.B. Methyl, oder Niederalkoxy, z.B. Methoxy, substituiertem Benzylalkohol, oder Phenolen, die gegebenenfalls durch geeignete Substituenten aktiviert sind, z.B. durch Halogen, etwa 4-Halogen, wie 4-Chlor, Niederalkoxy, etwa 4-Niederalkoxy wie 4-Methoxy, 4-Nitro oder 2,4-Dinitro, wie etwa 4-Chlorphenol, 4-Methoxyphenol, 4-Nitro- oder 2,4-Dinitrophenol, ferner Ester mit Cycloalkanolen, wie etwa Cyclopentanol oder Cyclohexanol, die gegebenenfalls durch Niederalkyl, z.B. Methyl, substituiert sein können. Die Umsetzung wird in an sich bekannter Weise, üblicherweise in Gegenwart eines inerten Lösungsmittels, z.B. in einem Temperaturbereich von etwa -10° bis +50°C in einem geschlossenen Gefäss durchgeführt.

Die Ausgangsstoffe der Formel VI, worin m für 0 steht, lassen sich in an sich bekannter Weise erhalten, indem man eine Verbindung der Formel

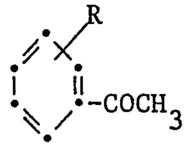

(VIa)

bromiert, die Carbonylgruppe in der so erhaltenen entsprechenden Halogenacetyl-Verbindung zur Carbinolgruppe, z.B. mittels Diboran, reduziert und die erhaltene Verbindung mit einem Amin der Formel

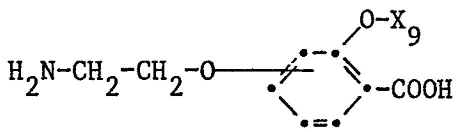

(VIb),

worin $X_9$ die angegebene Bedeutung hat, oder einem reaktionsfähigen Derivat davon, umsetzt. Man kann auch die genannte Halogenacetyl-Verbindung mit dem Amin der Formel VIb umsetzen und nachträglich die Carbonyl- in die Carbinolgruppe umwandeln.

Für die Herstellung von Ausgangsstoffen der Formel VI, worin m obige Bedeutung hat, kann man ferner die durch Umsetzung einer Verbindung der Formel

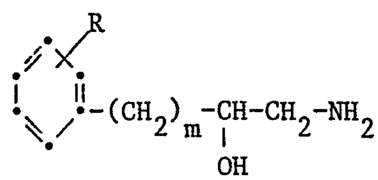

(VIc)

mit einer Carbonylverbindung der Formel

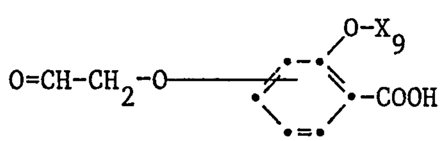

(VId)

gebildete Schiff'sche Base mit einem Borhydrid, etwa Natriumborhydrid reduzieren.

Die Reduktion kann auch mittels aktiviertem Wasserstoff in Gegenwart eines Hydrierkatalysators, z.B. eines Platin-auf-Kohle-Katalysators erfolgen.

Verbindungen der Formel VIc ihrerseits sind z.B. durch Umsetzung einer Verbindung der Formel

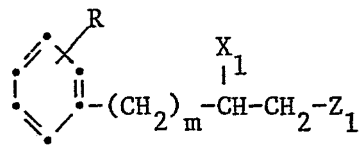 (VIe),

worin $X_1$ und $Z_1$ zusammen Epoxy, oder $X_1$ Hydroxy und $Z_1$ eine reaktionsfähige veresterte Hydroxygruppe, z.B. Halogen, wie Chlor, bedeutet, mit Ammoniak, oder, falls $X_1$ Hydroxy und $Z_1$ z.B. Chlor bedeutet, mit Hexamethylentetramin und Zersetzung des gebildeten Addukts mit verdünnter Mineralsäure, wie verdünnter Salzsäure, erhältlich, wobei Ausgangsstoffe der Formel VIe analog dem für die Herstellung von Ausgangsstoffen der Formel II beschriebenen Verfahren hergestellt werden können.

Carbonylverbindungen der Formel (VId) wiederum können durch Umsetzung einer Verbindung der Formel

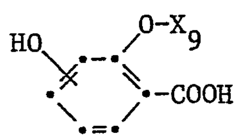 (VIf)

mit Chloracetaldehyd (IVf) in an sich bekannter Weise erhalten werden.

Die neuen Verbindungen der Formel I können ebenfalls erhalten werden, indem man in einer Verbindung der Formel

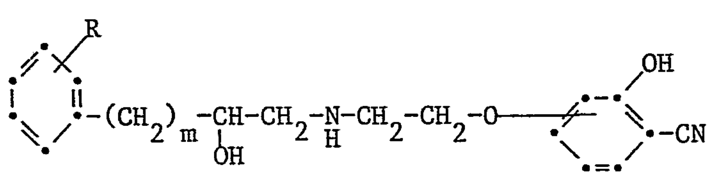 (VII),

worin eine oder beide Hydroxygruppen gegebenenfalls durch solche mittels Hydrolyse abspaltbaren und durch Wasserstoff ersetzbaren Gruppen geschützt sind, die unter den Bedingungen des Verfahrens abgespalten und der Wasserstoff ersetzt werden, die Gruppe-CN mittels Hydrolyse in die Gruppe $-CONH_2$ umwandelt, und, wenn erwünscht, die anschliessend an das erste Verfahren genannten zusätzlichen Verfahrensschritte durchführt.

Die Ausgangsstoffe der Formel VII lassen sich in üblicher Weise herstellen, indem man eine Verbindung der Formel

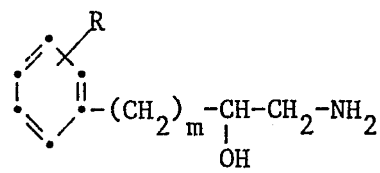

(VIIa)

mit einer Verbindung der Formel

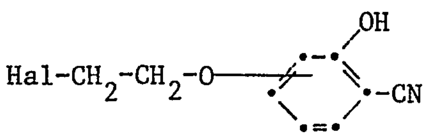

(VIIb),

worin Hal für Chlor, Brom oder Jod steht, umsetzt. Die Umsetzung wird vorteilhafterweise in Gegenwart eines basischen Mittels in an sich bekannter Weise durchgeführt.

Ausgangsstoffe der Formel (VIIa) ihrerseits, worin m obige Bedeutung hat, sind durch Umsetzung einer Verbindung der Formel (II), worin $X_1$ und $Z_1$ zudammen die Epoxygruppe bedeuten, mit Ammoniak, oder wenn $X_1$ Hydroxy und $Z_1$ als reaktionsfähige veresterte Hydroxygruppe z.B. Halogen, wie Chlor, bedeutet, z.B. mit Hexamethylentetramin und anschliessender Umwandlung der erhaltenen Addukte mit verdünnter Mineralsäure, wie verdünnter Salzsäure, erhältlich.

Ausgangsstoffe der Formel (VII), worin m für 0 steht, lassen sich ferner in üblicher Weise herstellen, indem man z.B. eine Verbindung der Formel

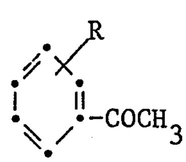

(VIIc)

halogeniert, z.B. bromiert, etwa mittels Brom in einem inerten Lösungsmittel, etwa Chloroform, in der so erhaltenen Halogenacetyl-, z.B. Bromacetyl-Verbindung das Halogen durch die Aminogruppe, z.B. durch Umsetzen mit Hexamethylentetramin in einem Lösungsmittel, etwa einem Chlorkohlenwasserstoff, wie Chloroform,und Zersetzen des erhaltenen Addukts mit verdünnter Mineralsäure, z.B. Salzsäure, ersetzt, in der erhaltenen Verbindung der Formel

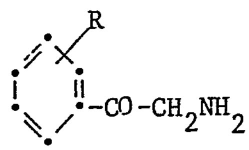 (VIId)

die Carbonylgruppe zur Carbinolgruppe, z.B. mittels Diboran, reduziert und die erhaltene Verbindung der Formel

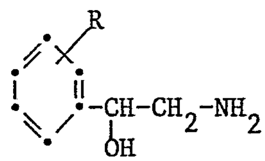 (VIIe)

mit einer Verbindung der oben erläuterten Formel VIIb in der dort angegebenen Weise umsetzt.

Eine Verbindung der Formel VIIb wiederum kann durch Einwirken von Essigsäureanhydrid auf das dem Cyanid entsprechende Oxim erhalten werden. Dies geschieht zweckmässigerweise durch Kochen unter Rückfluss. Das Oxim kann seinerseits aus dem entsprechenden Aldehyd durch Kochen mit Hydroxylamin-hydrochlorid in Gegenwart von alkoholischer Natriumcarbonatlösung unter Rückfluss hergestellt werden. Der entsprechende Aldehyd wiederum kann durch Umsetzung von 2,4-Dihydroxybenzaldehyd mit einem α,β-, α,γ- oder α,ω-Dihalogenniederalkan, vorzugsweise in Gegenwart eines basischen Mittels, hergestellt werden. Man kann aber auch in analoger Weise ein Hydroxysalicylonitril, z.B. das 2,4-Dihydroxybenzonitril [Chem.Ber. 24, 3657 (1891)] oder das 2,5-Dihydroxybenzonitril [Helv.Chim.Acta 30, 149, 153 (1947)] mit einem Dihalogenäthan zu einer Verbindung der Formel VIIb umsetzen.

Die neuen Verbindungen der Formel I, worin m für 3 steht, können ebenfalls erhalten werden, indem man in einer Verbindung der Formel

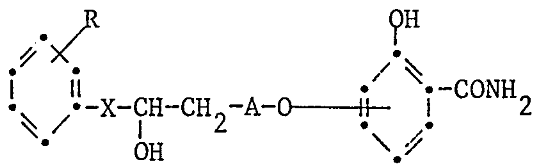 (VIII),

worin A für die Gruppe $-\underset{H}{N}-CH_2-CH_2-$ oder $-N{=}CH-CH_2$ steht, X eine mittels Reduktion, einschliesslich Hydrogenolyse, in eine 1,3-Propylengruppe überführbare Gruppe darstellt, und eine oder beide Hydroxygruppen durch solche mittels Reduktion, einschliesslich Hydrogenolyse, abspaltbaren und durch Wasserstoff ersetzbaren Gruppen geschützt sind, die unter den Bedingungen des Verfahrens abgespalten und durch Wasserstoff ersetzt werden, die Gruppe X mittels Reduktion einschliesslich Hydrogenolyse in eine 1,3-Propylengruppe und gleichzeitig eine gegebenenfalls vorhandene ungesättigte Gruppe A in die gesättigte Gruppe A umwandelt, und, wenn erwünscht, die anschliessend an das erste Verfahren genannten zusätzlichen Verfahrensschritte durchführt.

Eine Gruppe X stellt insbesondere eine anstelle einer Methylengruppe stehende Carbonylgruppe dar, die entsprechend der Bedeutung von m als 3 zusammen mit zwei Methylengruppen jede der möglichen Stellungen einnimmt. Eine Gruppe X stellt ausserdem eine zwei- oder dreifach ungesättigte Gruppe mit 3 Kohlenstoffatomen, z.B. eine 1,2-Propenylen-, 2,3-Propenylen- oder 1,3-Propinylengruppe dar.

Die Reduktion der Gruppe X erfolgt in üblicher Weise z.B. mittels aktiviertem Wasserstoff, etwa Wasserstoff in Gegenwart eines Hydrierkatalysators, z.N. eines Nickel- oder gegebenenfalls auf einem Trägermaterial, wie Aktivkohle, niedergeschlagenen Edelmetallkatalysators wie Platin oder Palladium, oder eines Platin- oder Palladium-auf-Kohle-Katalysators. Hierbei werden gegebenenfalls vorhandene hydrogenolytisch abspaltbare Hydroxyschutzgruppen abgespalten und durch Wasserstoff ersetzt. Die Reduktion, einschliesslich Hydrogenolyse, wird in an sich bekannter Weise, üblicherweise in Gegenwart eines inerten Lösungsmittels, bei normalem oder erhöhtem Druck und normaler oder erhöhter Temperatur vorgenommen.

Ausgangsstoffe der Formel VIII, worin X eine Gruppe der Formel $-CO-CH_2-CH_2-$ (VIIIa) bedeutet, können in an sich bekannter Weise erhalten werden, indem man in einer Verbindung der Formel

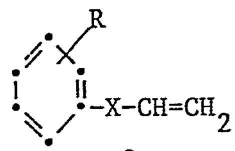 (IX)

die Gruppe $-CH=CH_2$ zur Gruppe $-\overset{O}{\widehat{CH - CH_2}}$ epoxidiert, (vgl. hierzu: Cahnmann, Bull.Soc.Chim.France [5], 4, 1937, 226, 230.) z.B., wie unter den Ausgangsstoffen der Formel (II) beschrieben, durch Umsetzung mit einer Peroxy-Verbindung, wie Wasserstoffperoxid, oder einer organischen Persäure, wie z.B. einer gegebenenfalls substituierten, wie halogenierten aliphatischen oder aromatischen Persäure, z.B. Peressigsäure oder Trifluorperessigsäure, Perbenzoesäure oder m-Chlorperbenzoesäure in einem wasserfreien Lösungsmittel, z.B. Chloroform oder Dioxan. Anschliessend kann man die erhaltene Verbindung oder eine hieraus z.B. durch Umsetzung mit einer Halogenwasserstoffsäure, z.B. Chlorwasserstoffsäure in einem Lösungsmittel, wie Dioxan, erhaltene Verbindung der allgemeinen Formel

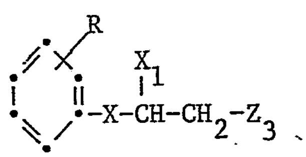 (X),

worin $X_1$ Hydroxy und $Z_3$ Halogen, z.B. Chlor, bedeutet, mit Ammoniak oder mit Hexamethylentetramin umsetzen und anschliessend das erhaltene Addukt durch Zersetzen mit einer verdünnten Mineralsäure, z.B. Salzsäure, in eine Verbindung der Formel

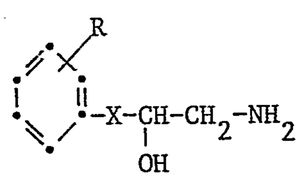 (XI)

umwandeln. Aus dieser lässt sich durch Umsetzung mit einer Verbindung der Formel

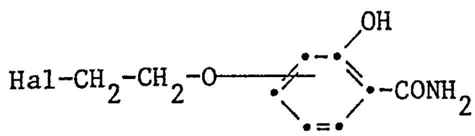

(XII),

worin Hal für Halogen und insbesondere für Chlor steht, ein Ausgangsmaterial der Formel VIII mit der Gruppe A der Formel $-\underset{H}{N}-CH_2-CH_2-$, oder durch Umsetzung mit einer Verbindung der Formel

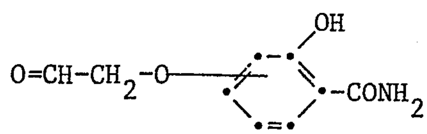

(XIII),

ein Ausgangsmaterial der Formel VIII, worin die Gruppe A der Formel $-N=CH-CH_2-$ entspricht, herstellen. Diese Umsetzungen werden in an sich bekannter Weise vorgenommen, z.B. verwendet man bei der Umsetzung mit einer Verbindung der Formel XII ein alkalisches Kondensationsmittel, z.B. ein Alkali- oder Erdalkalicarbonat, wie Natrium- bzw. Calciumcarbonat.

Ausgangsstoffe der Formel VIII, worin X eine zwei- oder dreifach ungesättigte Gruppe mit 3 Kohlenstoffatomen bedeutet, und A die angegebene Bedeutung hat, können erhalten werden, indem man eine Verbindung der Formel

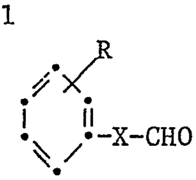

(XIV),

worin X eine zwei- oder dreifach ungesättigte Gruppe mit 3 Kohlenstoffatomen bedeutet, oder ein geeignetes Derivat davon, etwa ein Acetal, z.B. das Dimethylacetal, oder eine Bisulfitadditionsverbindung, mit einer geeigneten Silicium-organischen Cyanverbindung, etwa einem Triniederalkyl-, z.B. Trimethylsilylcyanid, in üblicher Weise in Gegenwart eines Katalysators, wie Zinkjodid, in eine Verbindung der Formel

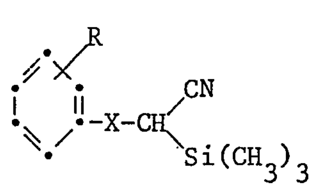

(XV)

umwandelt, und in dieser die Gruppe -CN mittels Reduktion, unter gleichzeitigem Ersatz der Trimethylsil,lgruppe durch Hydroxy, in die Gruppe $-CH_2NH_2$ überführt. Als Reduktionsmittel kann man z.B. ein Dileichtmetallhydrid, etwa Lithiumaluminiumhydrid, in einem geeigneten Lösungsmittel verwenden, wozu üblicherweise eine ätherartige Flüssigkeit, z.B. Diäthyläther oder Tetrahydrofuran, dient.

Die hiernach erhaltene Verbindung der Formel

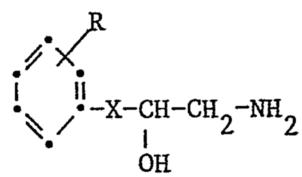

(XVI)

kann man anschliessend mit einer Verbindung der oben erläuterten Formel XII zu einer Verbindung der Formel (VIII) umsetzen, worin A die Gruppe der Formel $-\underset{H}{N}-CH_2-CH_2-$ darstellt; oder man setzt eine Verbindung der Formel XVI mit einer Verbindung der oben erläuterten Formel (XIII) um, wonach ein Ausgangsmaterial der Formel VIII erhalten wird, worin A die Gruppe der Formel $-N=CH-CH_2-$ bedeutet.

Diese Umsetzungen werden in üblicher Weise durchgeführt.

Bei der Auswahl des geeigneten obigen Verfahrens zur Herstellung von Verbindungen der Formel I muss darauf geachtet werden, dass vorhandene Substituenten, in erster Linie in dem durch R substituierten aromatischen Rest, nicht umgewandelt oder abgespalten werden.

Je nach den Verfahrensbedingungen und Ausgangsstoffen erhält man die neuen Verbindungen in freier Form oder in der ebenfalls von der Erfindung umfassten Form ihrer Salze, wobei die neuen Verbindungen oder Salze davon auch als Hemi-, Mono-, Sesqui- oder Polyhydrate davon vorliegen können. Säureadditionssalze der neuen Verbindungen können in an sich bekannter Weise, z.B. durch Behandeln mit basischen Mitteln, wie Alkalimetallhydroxiden, -carbonaten oder -hydrogencarbonaten oder Ionenaustauschern, in die freien Verbindungen übergeführt werden.

Andererseits können erhaltene freie Basen mit organischen oder anorganischen Säuren, z.B. mit den genannten Säuren, Säureadditionssalze bilden, wobei zu deren Herstellung insbesondere solche Säuren verwendet werden, die sich zur Bildung von pharmazeutisch annehmbaren Salzen eignen.

Diese oder andere Salze, insbesondere Säureadditionssalze der neuen Verbindungen, wie z.B. Oxalate oder Perchlorate, können auch zur Reinigung der erhaltenen freien Basen dienen, indem man die freien Basen in Salze überführt, diese abtrennt und reinigt, und aus den Salzen wiederum die Basen freisetzt.

Die neuen Verbindungen können je nach der Wahl der Ausgangsstoffe und Arbeitsweisen, als optische Antipoden oder Racemate vorliegen. Die Ausgangsstoffe können auch als bestimmte optische Antipoden eingesetzt werden.

Erhaltene Racemate lassen sich nach an sich bekannten Methoden in die Antipoden zerlegen, z.B. durch Umkristallisieren aus einem optisch aktiven Lösungsmittel, durch Behandeln mit geeigneten Mikroorganismen oder durch Umsetzen mit einer, mit der racemischen Verbindung Salze bildenden optisch aktiven Substanz, insbesondere Säuren, und Trennen des auf diese Weise erhaltenen Salzgemisches, z.B. auf Grund von verschiedenen Löslichkeiten, in die diastereomeren Salze, aus denen die freien Antipoden durch Einwirkung geeigneter Mittel freigesetzt werden können. Besonders gebräuchliche, optisch aktive Säuren sind z.B. die D- und L-Formen von Weinsäure, Di-O,O'-(p-Toluoyl)-weinsäure, Aepfelsäure, Mandelsäure, Camphersulfonsäure, Glutaminsäure, Asparaginsäure oder Chinasäure. Vorteilhaft isoliert man den wirksameren der beiden Antipoden.

Die Erfindung betrifft auch diejenigen Ausführungsformen des Verfahrens, nach denen man von einer auf irgendeiner Stufe des Verfahrens als

Zwischenprodukt erhältlichen Verbindung ausgeht und die fehlenden Verfahrensschritte durchführt, oder das Verfahren auf irgendeiner Stufe abbricht, oder bei denen man einen Ausgangsstoff unter den Reaktionsbedingungen bildet, oder bei denen eine Reaktionskomponente gegebenenfalls in Form ihrer Salze vorliegt.

Die Ausgangsstoffe sind bekannt oder können, falls sie neu sind, nach an sich bekannten Methoden, wie oben, z.B. analog wie in den Beispielen beschrieben, erhalten werden. Neue Ausgangsstoffe bilden ebenfalls einen Gegenstand der Erfindung. Die Erfindung betrifft auch verfahrensgemäss erhältliche Zwischenprodukte.

Die neuen Verbindungen können z.B. in Form pharmazeutischer Präparate Verwendung finden, welche eine pharmakologisch wirksame Menge der Aktivsubstanz, gegebenenfalls zusammen mit pharmazeutisch verwendbaren Trägerstoffen, enthalten, die sich zur enteralen, z.B. oralen, oder parenteralen Verabreichung eignen, und anorganisch oder organisch, fest oder flüssig sein können. So verwendet man Tabletten oder Gelatinekapseln, welche den Wirkstoff zusammen mit Verdünnungsmitteln, z.B. Laktose, Dextrose, Sukrose, Mannitol, Sorbitol, Cellulose und/oder Glycerin und/oder Schmiermitteln, z.B. Kieselerde, Talk, Stearinsäure oder Salze davon, wie Magnesium- oder Calciumstearat, und/oder Polyäthylenglykol, aufweisen. Tabletten können ebenfalls Bindemittel, z.B. Magnesiumaluminiumsilikat, Stärken, wie Mais-, Weizen-, Reis- oder Pfeilwurzelstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel enthalten. Ferner kann man die neuen pharmakologisch wirksamen Verbindungen in Form von parenteral verabreichbaren Präparaten oder von Infusionslösungen verwenden. Solche Lösungen sind vorzugsweise isotonische Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, in an sich bekannter Weise, z.B. mittels konventioneller Misch-, Granulier-, Dragier-, Lösungs- oder Lyophilisierungsverfahren hergestellt werden, enthalten von etwa 0,1% bis 100%, insbesondere von etwa 1% bis etwa 50%, Lyophilisate bis zu 100% des Aktivstoffes.

Die Dosierung kann von verschiedenen Faktoren, wie Applikationsweise, Spezies, Alter und/oder individuellem Zustand abhängen. So liegen die täglich in einer oder mehreren, vorzugsweise höchstens 4 Einzeldosen zu verabreichenden Dosen bei oraler Applikation an Warmblüter von etwa 70 kg Körpergewicht zur Behandlung von Depressionen in einem Bereich von etwa 5-200 mg, für β-Rezeptorenblocker vorzugsweise zwischen etwa 0,02 g und etwa 0,2 g, und für β-Rezeptoren-Stimulatoren zwischen etwa 0,002 g und etwa 0,04 g.

Die folgenden Beispiele dienen zur Illustration der Erfindung; Temperaturen werden in Celsiusgraden angegeben.

<u>Beispiel 1:</u> Eine Lösung von 25 g rohem α-[N-[2-(3-Carbamoyl-4-hydroxy-phenoxy)-äthyl]-benzylaminomethyl]-4-fluor-benzylalkohol in 200 ml Methanol wird nach Zusatz von 2 g Palladium-auf-Kohle-Katalysator (5%-ig) bis zur Aufnahme der berechneten Menge Wasserstoff hydriert. Durch Filtration und Eindampfen des Filtrats erhält man einen dickflüssigen Rückstand, aus dem durch Lösen in Methanol und Abkühlen der α-[N-[2-(3-Carbamoyl-4-hydroxy-phenoxy)-äthyl]-aminomethyl]-4-fluor-benzylalkohol auskristallisiert, welcher nach dem Umkristallisieren aus Dioxan den Smp. 186-187° zeigt.

Das Ausgangsmaterial kann auf folgende Weise hergestellt werden:

1a) Eine Lösung von 8,3 g 1-(4-Fluor-phenyl)-oxiran und 14,3 g 5-[(2-Benzylamino)-äthoxy]-salicylamid in 100 ml Isopropanol wird 18 Stunden unter Rückfluss erhitzt und dann eingedampft. Der so als oranges Oel erhaltene α-[N-[2-(3-Carbamoyl-4-hydroxy-phenoxy)-äthyl]-benzylamino-methyl]-4-fluor-benzylalkohol wird als solcher weiterverarbeitet.

Beispiel 2: Analog den in den Beispielen 1a) und 1) beschriebenen Arbeitsweisen erhält man unter Verwendung von:
a) 1-(2-Methyl-phenyl)-oxiran den α-[N-[2-(3-Carbamoyl-4-hydroxy-phenoxy)-äthyl]-aminomethyl]-2-methyl-benzylalkohol vom Smp. 151-152° (je einmal umkristallisiert aus Aethylacetat bzw. Dioxan).

b) 1-(3-Methyl-phenyl)-oxiran den α-[N-[2-(3-Carbamoyl-4-hydroxy-phenoxy)-äthyl]-aminomethyl]-3-methyl-benzylalkohol vom Smp. 180-182° (aus Dioxan).

c) 1-(4-Methyl-phenyl)-oxiran den α-[N-[2-(3-Carbamoyl-4-hydroxy-phenoxy)-äthyl]-aminomethyl]-4-methyl-benzylalkohol vom Smp. 190-191° (aus Dioxan).

Beispiel 3: 18,7 g 5-(2-Amino-äthoxy)-salicylamid werden in 100 ml Dimethylsulfoxid durch Erwärmen gelöst. Wenn die Temperatur der Lösung 80-85° erreicht hat, werden 18,4 g 1-(4-Chlor-phenyl)-oxiran auf einmal zugegeben und das Reaktionsgemisch 45 Minuten bei 80-85° gerührt. Hierauf wird die Lösung auf 1500 ml Wasser gegossen und nach Zusatz von 50 ml Aethylacetat 30 Minuten gerührt. Die ausgefallenen Kristalle werden abgesaugt und aus Methanol umkristallisiert. Nach dem Absaugen und Trocknen der Kristalle erhält man den α-[N-[2-(3-Carbamoyl-4-hydroxy-phenoxy)-äthyl]-aminomethyl]-4-chlor-benzylalkohol vom Smp. 189-190°.

Beispiel 4: Analog Beispiel 3 erhält man unter Verwendung von 23,7 g 1-(4-Brom-phenyl)-oxiran den α-[N-[2-(3-Carbamoyl-4-hydroxy-phenoxy)-äthyl]-aminomethyl]-4-brom-benzylalkohol vom Smp. 189-190°.

Beispiel 5: Die Lösung von 28 g rohem α-[N-[2-(4-Benzyloxy-3-carbamoyl-phenoxy)-äthyl]-benzylaminomethyl]-4-methoxy-benzylalkohol in 300 ml Methanol wird unter Zusatz von 4,0 g Palladium-auf-Kohle-Katalysator (10 %-ig) bei Normalbedingungen bis zum Stillstand unter Aufnahme von 2-Mol-Aequivalenten Wasserstoff hydriert. Das bereits auskristallisierte Produkt wird durch Zusatz von Dioxan und Erwärmen gelöst, der Katalysator abfiltriert und das Filtrat eingedampft. Die erhaltenen Kristalle werden aus Dioxan umkristallisiert und ergeben den α-[N-[2-(3-Carbamoyl-4-hydroxy-phenoxy)-äthyl]-aminomethyl]-4-methoxy-benzylalkohol vom Smp. 183-184°.

Das Ausgangsmaterial wird auf folgende Weise hergestellt:

5a) Ein Gemisch von 18,8 g 5-(2-Benzylamino-äthoxy)-2-benzyloxy-benzamid, 13,8 g ω-Brom-4-methoxy-acetophenon und 10,5 g Kaliumcarbonat in 200 ml Dioxan wird 20-24 Stunden bei Raumtemperatur gerührt. Die Suspension wird filtriert und das Filtrat eingedampft. Das so erhaltene Oel wird in 250 ml Methanol gelöst und hierin im Verlaufe von ca. 2 Stunden unter Rühren portionenweise 9,0 g Natriumborhydrid eingetragen, wobei durch Kühlen die Temperatur bei 20-30° gehalten wird. Das Gemisch wird eine weitere Stunde gerührt, hierauf eingedampft und der Eindampfrückstand zwischen Aethylacetat und Wasser verteilt. Die organische Phase wird mit Wasser gewaschen, über Magnesiumsulfat getrocknet und eingedampft. Man erhält den rohen α-[N-[2-(4-Benzyloxy-3-carbamoyl-phenoxy)-äthyl]-benzylaminomethyl]-4-methoxy-benzylalkohol als Oel, das kristallin erstarrt und als solches weiterverwendet wird.

Beispiel 6: Eine Lösung von 25 g rohem α-[N-[2-(4-Carbamoyl-3-hydroxy-phenoxy)-äthyl]-benzylaminomethyl]-4-methyl-benzylalkohol in 400 ml Methanol wird unter Zusatz von 3,0 g Palladium-auf-Kohle-Katalysator (10 %-ig) bis zur Aufnahme von 1 Mol-Aequivalent Wasserstoff hydriert und analog Beispiel 5 aufgearbeitet. Man erhält den α-[N-[2-(4-Carbamoyl-3-hydroxy-phenoxy)-äthyl]-aminomethyl]-4-methyl-benzylalkohol

vom Smp. 160-165°. Durch Umsetzen mit der berechneten Menge Salzsäure in Methanol erhält man das Hydrochlorid vom Smp. 243-244°.

Das Ausgangsmaterial wird auf folgende Weise hergestellt:

6a) Eine Lösung von 10 g 1-(4-Methyl-phenyl)-oxiran in 100 ml Isopropanol wird mit 12,0 g 4-(2-Benzylamino-äthoxy)-salicylamid 48 Stunden unter Rückfluss gekocht. Durch Eindampfen erhält man rohes α-[N-[2-(4-Carbamoyl-3-hydroxy-phenoxy)-äthyl]-benzylaminomethyl]-4-methylbenzylalkohol als Oel, der ohne weitere Reinigung weiterverarbeitet wird.

Beispiel 7: Eine Lösung von 11,1 g 1-[N-Benzyl-2-(3-carbamoyl-4-hydroxy-phenoxy)-äthylamino]-5-(2-methoxyphenyl)-2-pentanol in 100 ml Methanol wird unter Zusatz von 1 g Palladium-auf-Kohle-Katalysator (5%-ig) unter Normalbedingungen bis zur beendeten Wasseraufnahme hydriert. Der Katalysator wird abfiltriert, das Filtrat am Rotationsverdampfer vom Lösungsmittel befreit und der Rückstand aus Isopropanol umkristallisiert, wonach man das 1-[2-(3-Carbamoyl-4-hydroxy-phenoxy)-äthylamino]-5-(2-methoxyphenyl)-2-pentanol erhält. Durch Behandeln mit überschüssiger methanolischer Salzsäure, Entfernen des Lösungsmittels am Rotationsverdampfer und Umkristallisieren des Rückstandes aus Acetonitril-Aether erhält man das Hydrochlorid vom Smp. 110-112°.

Das als Ausgangsmaterial benötigte 1-[N-Benzyl-2-(3-carbamoyl-4-hydroxy-phenoxy)-äthylamino]-5-(2-methoxyphenyl)-2-pentanol kann wie folgt hergestellt werden:

7a) 24,3 g mit Chloroform gewaschene Magnesiumspäne werden unter Stickstoff in 50 ml Aether suspendiert und mit einigen Tropfen einer Lösung von 56,0 g 4-Brom-1-buten in 40 ml Aether versetzt. Nach dem Einsetzen der Reaktion schaltet man den Rührer ein, versetzt vorerst mit 200 ml Aether, kühlt das Reaktionsgemisch im Eisbad und

lässt im Verlaufe von 4 Stunden den Rest der Brombutenlösung langsam zulaufen. Das Reaktionsgemisch wird während 30 Minuten im Eisbad nachgerührt und die trübe Lösung dann unter Stickstoff durch Glaswatte in einen zweiten Rührkolben filtriert. Zu dieser Lösung wird nun im Verlaufe von 30 Minuten eine Lösung von 34,2 g 2-Methoxy-benzylbromid in 60 ml Toluol bei 15-20° zugetropft. Das Reaktionsgemisch wird 15 Stunden bei Raumtemperatur und 1 Stunde unter Rückfluss nachgerührt, anschliessend im Eisbad abgekühlt, tropfenweise mit 20 ml Wasser versetzt, die entstehende Suspension durch ein Filterhilfsmittel filtriert, mit Aether nachgewaschen und das Filtrat 3 mal mit 0,1-n. Salzsäure extrahiert. Nach dem Trocknen der organischen Phase und Entfernen des Lösungsmittels am Rotationsverdampfer wird der Rückstand im Vakuum destilliert, wobei man das 1-(4-Pentenyl)-2-methoxybenzol erhält; Kp.: 54-60°/0,03 Torr.

7b) Zu einer Lösung von 9,7 g 1-(4-Pentenyl)-2-methoxybenzol in 50 ml Methylenchlorid wird unter Rühren bei Raumtemperatur eine Lösung von 13,0 g m-Chlorperbenzoesäure (85%-ig) in 120 ml Methylenchlorid langsam zugetropft. Nach 4 Stunden wird abfiltriert, das Filtrat mit 10%-iger Natriumsulfitlösung gewaschen, über Natriumsulfat getrocknet und eingeengt. Das zurückbleibende rohe 1-(4,5-Epoxypentyl)-2-methoxybenzol wird als solches weiterverarbeitet.

7c) 6 g rohes 1-(4,5-Epoxypentyl)-2-methoxybenzol und 8,1 g 4-[2-(Benzylamino)-äthoxy]-salicylamid werden in 150 ml Isopropanol gelöst und die Lösung während 48 Stunden unter Rückfluss erhitzt. Anschliessend wird das Lösungsmittel entfernt und der Rückstand über 450 g Kieselgel mit einem Gemisch von Chloroform/Methanol (95/5) chromatographiert, wobei man aus den Hauptfraktionen das 1-[N-Benzyl-2-(3-carbamoyl-4-hydroxy-phenoxy)-äthylamino]-5-(2-methoxyphenyl)-2-pentanol als Oel erhält, welches als solches weiterverarbeitet wird.

Beispiel 8: Eine Lösung von 3,0 g α-Aminomethyl-4-methylbenzylalkohol und 4,8 g (2,3-Dihydroxy-2,2-dimethyl-4H-1,3-benzoxazin-4-on-6-yloxy)-acetaldehyd in 150 ml Methanol wird mit einer Lösung von Chlorwasserstoff in Methanol neutralisiert (pH ca. 6), mit 10 g Molekularsieb (3 Å) und hierauf mit 6,2 g Natriumcyanborhydrid versetzt. Die Suspension wird über Nacht bei einer Temperatur von 20-25° gerührt, filtriert, mit verdünnter Salzsäure unter Eiskühlung zersetzt und im Vakuum eingedampft. Der Eindampfrückstand wird in Wasser gelöst, die Lösung klarfiltriert und das Filtrat mit konz. Ammoniak alkalisch eingestellt (pH 8-9), wonach der α-[N-[2-(3-Carbamoyl-4-hydroxy-phenoxy)-äthyl]-aminomethyl]-4-methyl-benzylalkohol allmählich auskristallisiert, der nach zweimaliger Umkristallisation aus Dioxan den Smp. 190-191° zeigt.

Beispiel 9: 4,5 g α-[N-[2-(3-Carbomethoxy-4-hydroxy-phenoxy)-äthyl]-aminomethyl]-4-methyl-benzylalkohol werden in 20 ml Dioxan gelöst, mit 100 ml konz. Ammoniak versetzt und die Lösung 3-4 Tage bei Raumtemperatur verschlossen stehen gelassen. Durch Eindampfen der Lösung und Umkristallisation des Eindampfrückstandes aus Dioxan erhält man α-[N-[2-(3-Carbamoyl-4-hydroxyphenoxy)-äthyl]-aminomethyl]-4-methyl-benzylalkohol vom Smp. 190-191°.

Das Ausgangsmaterial kann auf folgende Weise hergestellt werden:

a) Ein Gemisch von 13,4 g 2,5-Dihydroxybenzoesäure-methylester, 12,0 g Kaliumcarbonat und 80 g 1,2-Dibromäthan wird unter Rühren 35 Stunden unter Rückfluss zum Sieden erhitzt. Das Reaktionsgemisch wird filtriert, das Filtrat im Vakuum eingedampft und der Eindampfrückstand in 200 ml Aethylacetat gelöst. Durch zweimaliges Waschen der organischen Phase mit Wasser (jeweils 50 ml), Trocknen über Magnesiumsulfat und Eindampfen der Lösung im Vakuum erhält man rohen 5-(2-Bromäthoxy)-salicylsäuremethylester als Oel, das allmählich kristallisiert; Smp. 53-55° (aus Isopropanol).

b) Ein Gemisch von 6,5 g α-(Aminomethyl)-4-methyl-benzylalkohol, 7,7 g 5-(2-Bromäthoxy)-salicylsäuremethylester und 4,2 g Kaliumcarbonat wird unter Rühren in einem Bad von 120-130° geschmolzen. Nach einer halben Stunde wird das Reaktionsgemisch abgekühlt und zwischen 50 ml Wasser und 100 ml Aethylacetat verteilt. Die organische Phase wird abgetrennt, mit 50 ml Wasser gewaschen, über Magnesiumsulfat getrocknet und eingedampft, wonach man α-[N-[2-(3-Carbomethoxy-4-hydroxy-phenoxy)-äthyl]-aminomethyl]-4-methyl-benzylalkohol als Oel erhält, welches ohne weitere Reinigung weiterverarbeitet wird.

Beispiel 10: Tabletten enthaltend 20 mg an aktiver Substanz werden in folgender Zusammensetzung in üblicher Weise hergestellt:

Zusammensetzung:

| | |
|---|---|
| α-[N-[2-(3-Carbamoyl-4-hydroxy-phenoxy)-äthyl]-aminomethyl]-4-chlor-benzylalkohol | 20 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 145 mg |

Herstellung:

α-[N-[2-(3-Carbamoyl-4-hydroxy-phenoxy)-äthyl]-aminomethyl]-4-chlor-benzylalkohol wird mit einem Teil der Weizenstärke, mit Milchzucker und kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist.

Die plastische Masse wird durch ein Sieb von ca. 3 mm Maschenweite gedrückt, getrocknet und das erhaltene trockene Granulat nochmals durch ein Sieb getrieben. Darauf werden die restlichen Weizenstärke, Talk und Magnesiumstearat zugemischt und die Mischung zu Tabletten von 145 mg Gewicht mit Bruchkerbe verpresst.

Beispiel 11: Tabletten enthaltend 1 mg an aktiver Substanz werden in folgender Zusammensetzung in üblicher Weise hergestellt:

Zusammensetzung:

| | |
|---|---|
| α-[N-[2-(3-Hydroxy-4-carbamoyl-phenoxy)-äthyl]-aminomethyl]-4-methyl-benzylalkohol | 1 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 126 mg |

Herstellung:

α-[N-[2-(3-Hydroxy-4-carbamoyl-phenoxy)-äthyl]-aminomethyl]-4-methyl-benzylalkohol wird mit einem Teil der Weizenstärke, mit Milchzucker und kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist.

Die plastische Masse wird durch ein Sieb von ca. 3 mm Maschenweite gedrückt, getrocknet und das erhaltene trockene Granulat nochmals durch ein Sieb getrieben. Darauf werden die restliche Weizenstärke, Talk und Magnesiumstearat zugemischt und die Mischung zu Tabletten von 126 mg Gewicht mit Bruchkerbe verpresst.

Beispiel 12: Kapseln enthaltend 10 mg an aktiver Substanz werden wie folgt auf übliche Weise hergestellt:

Zusammensetzung:

| | |
|---|---|
| α-[N-[2-(3-Carbamoyl-4-hydroxy-phenoxy)-äthyl]-aminomethyl]-4-fluor-benzylalkohol | 2500 mg |
| Talkum | 200 mg |
| Kolloidale Kieselsäure | 50 mg |

Herstellung:
Die aktive Substanz wird mit Talkum und kolloidaler Kieselsäure innig gemischt, das Gemisch durch ein Sieb mit 0,5 mm Maschenweite getrieben und dieses in Portionen von jeweils 11 mg in Hartgelatinekapseln geeigneter Grösse abgefüllt.

Beispiel 13: Eine sterile Lösung von 5,0 g α-[N-[2-(3-Carbamoyl-4-hydroxy-phenoxy)-äthyl]-aminomethyl]-2-methyl-benzylalkohol in 5000 ml destilliertem Wasser wird in Ampullen zu 5 ml abgefüllt, die in 5 ml Lösung 5 mg Wirkstoff enthalten.

Beispiel 14: 3,62 g α-[N-[2-(3-Carbamoyl-4-hydroxy-phenoxy)-äthyl]-aminomethyl]-3-methyl-benzylalkohol werden unter Zusatz von 100,0 ml 0,10-n. Salzsäure mit 18000 ml destilliertem Wasser auf ein Volumen von 18100 ml gelöst. Die sterilisierte Lösung wird in Ampullen à 5,0 ml abgefüllt, in denen 1 mg Wirkstoff enthalten ist.

Beispiel 15: Anstelle der in den Beispielen 10 bis 14 als aktive Substanz verwendeten Verbindungen können auch folgende Verbindungen der Formel I, oder deren pharmazeutisch annehmbaren nicht-toxischen Säureadditionssalze als Wirkstoffe in Tabletten, Dragées, Kapseln, Ampullenlösungen etc. verwendet werden:

α-[N-[2-(4-Carbamoyl-3-hydroxy-phenoxy)-äthyl]-aminomethyl]-4-methyl-benzylalkohol,

α-[N-[2-(3-Carbamoyl-4-hydroxy-phenoxy)-äthyl]-aminomethyl]-4-brom-benzylalkohol,

α-[N-[2-(3-Carbamoyl-4-hydroxy-phenoxy)-äthyl]-aminomethyl]-4-methoxy-benzylalkohol oder das

1-[2-(3-Carbamoyl-4-hydroxy-phenoxy)-äthylamino]-5-(2-methoxyphenyl)-2-pentanol.

Patentansprüche

1. Neue substituierte 2-Aminoäthanole der Formel

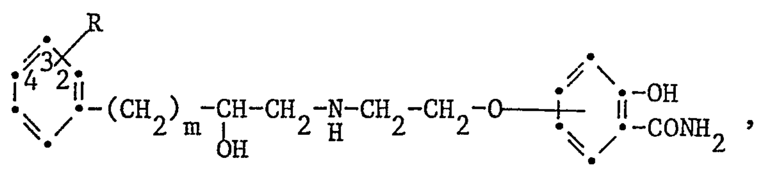

worin R Methyl, Methoxy, Fluor, Chlor oder Brom bedeutet, und m für Null oder 3 steht, mit der Massgabe, dass, wenn m Null ist, R für 2-Methyl, 3-Methyl, 4-Methyl, 4-Fluor, 4-Chlor, 4-Brom oder 4-Methoxy steht, und die basische Seitenkette über ihr Sauerstoffatom an den Salicylamidrest in 4-Stellung zur darin vorhandenen Hydroxygruppe gebunden ist, oder, wenn m Null ist und R für 4-Methyl steht, die basische Seitenkette über ihr Sauerstoffatom an den Salicylamidrest in 4-Stellung zur darin vorhandenen Carboxamidgruppe gebunden ist, oder, wenn m 3 bedeutet, R für 2-Methoxy steht und die basische Seitenkette über ihr Sauerstoffatom an den Salicylamidrest in 4-Stellung zur darin vorhandenen Hydroxygruppe gebunden ist, als Racemate oder optische Antipoden.

2. α-[N-[2-(3-Carbamoyl-4-hydroxy-phenoxy)-äthyl]-aminomethyl]-4-fluor-benzylalkohol.

3. α-[N-[2-(3-Carbamoyl-4-hydroxyphenoxy)-äthyl]-aminomethyl]-2-methyl-benzylalkohol.

4. α-[N-[2-(3-Carbamoyl-4-hydroxy-phenoxy)-äthyl]-aminomethyl]-3-methyl-benzylalkohol.

5. α-[N-[2-(3-Carbamoyl-4-hydroxy-phenoxy)-äthyl]-aminomethyl]-4-methyl-benzylalkohol.

6. α-[N-[2-(3-Carbamoyl-4-hydroxy-phenoxy)-äthyl]-aminomethyl]-4-chlor-benzylalkohol.

7. α-[N-[2-(3-Carbamoyl-4-hydroxy-phenoxy)-äthyl]-aminomethyl]-4-brom-benzylalkohol.

8. α-[N-[2-(3-Carbamoyl-4-hydroxy-phenoxy)-äthyl]-aminomethyl]-4-methoxy-benzylalkohol.

9. α-[N-[2-(4-Carbamoyl-3-hydroxy-phenoxy)-äthyl]-aminomethyl]-4-methyl-benzylalkohol.

10. 1-[2-(3-Carbamoyl-4-hydroxy-phenoxy)-äthylamino]-5-(2-methoxyphenyl)-2-pentanol.

11. Salze von Verbindungen der Ansprüche 1 bis 10.

12. Pharmazeutisch annehmbare, nicht-toxische Säureadditionssalze von Verbindungen der Ansprüche 1 bis 10.

13. Pharmazeutische Präparate enthaltend eine Verbindung der Ansprüche 1 bis 10 und 12 zusammen mit inerten Hilfs- und Trägerstoffen.

14. Verbindungen der Formel I zur Anwendung in einem Verfahren zur therapeutischen Behandlung des menschlichen oder tierischen Körpers.

15. Verbindungen der Formel I als Antidepressiva.

16. Verbindungen der Formel I als Blocker cardialer β-Rezeptoren.

17. Verbindungen der Formel I als Stimulatoren cardialer β-Rezeptoren.

18. Verwendung von Verbindungen der Formel I zur Herstellung von pharmazeutischen Präparaten.

19. Verwendung von Verbindungen der Formel I zur Bekämpfung von reaktiven oder endogenen Depressionen unterschiedlichen Schweregrades, sowie neurotischen oder anderen psychischen Störungen mit Antriebsschwäche und depressiven Verstimmungen.

20. Verwendung von Verbindungen der Formel I zur Bekämpfung von Rhythmusstörungen (Arrhythmien), Angina pectoris und der Hypertonie.

21. Verwendung von Verbindungen der Formel I zur Bekämpfung der insuffizienten Herzleistung sowie von Asthma und Durchblutungsstörungen.

22. Verfahren zur Herstellung von neuen substituierten 2-Aminoäthanolen der Formel

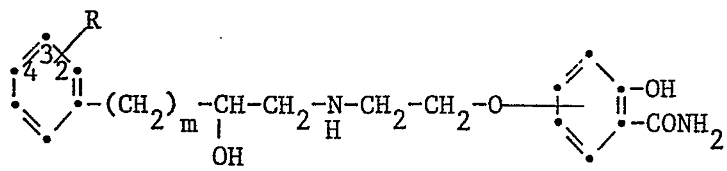

(I),

worin R Methyl, Methoxy, Fluor, Chlor oder Brom bedeutet, und m für Null oder 3 steht, mit der Massgabe, dass, wenn m Null ist, R für 2-Methyl, 3-Methyl, 4-Methyl, 4-Fluor, 4-Chlor, 4-Brom oder 4-Methoxy steht, und die basische Seitenkette über ihr Sauerstoffatom an den Salicylamidrest in 4-Stellung zur darin vorhandenen Hydroxygruppe gebunden ist, oder, wenn m Null ist und R für 4-Methyl steht, die basische Seitenkette über ihr Sauerstoffatom an den Salicylamidrest in 4-Stellung zur darin vorhandenen Carboxamidgruppe gebunden ist, oder, wenn m 3 bedeutet, R für 2-Methoxy steht und die basische Seitenkette über ihr Sauerstoffatom an den Salicylamidrest in 4-Stellung zur darin vorhandenen Hydroxygruppe gebunden ist, als Racemate oder optische Antipoden, dadurch gekennzeichnet, dass man

a) eine Verbindung der Formel

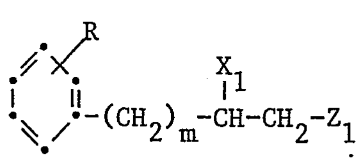

(II)

mit einer Verbindung der Formel

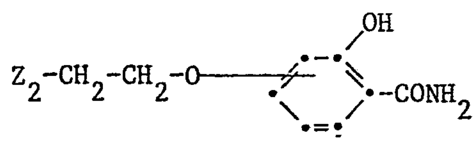 (III),

worin eine der Gruppen $Z_1$ und $Z_2$ eine reaktionsfähige veresterte Hydroxygruppe darstellt und die andere für die primäre Aminogruppe steht, und $X_1$ Hydroxy bedeutet, oder worin $X_1$ und $Z_1$ zusammen die Epoxygruppe bedeuten und $Z_2$ für die primäre Aminogruppe steht, R und m obige Bedeutung haben, umsetzt, oder

b) in einer Verbindung der Formel

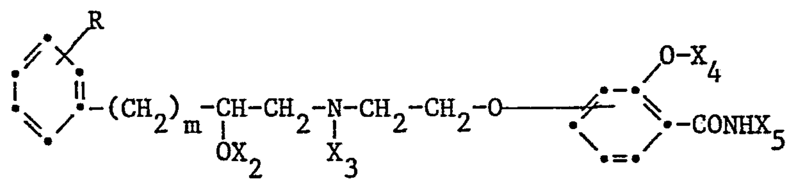 (IV),

worin $X_2$, $X_3$, $X_4$ und $X_5$ jeweils Wasserstoff oder einen durch Wasserstoff ersetzbaren Substituenten bedeuten, oder $X_2$ und $X_3$ und/oder $X_4$ und $X_5$ zusammen einen zweiwertigen, durch zwei Wasserstoffatome ersetzbaren Rest bedeuten mit der Massgabe, dass mindestens einer der Reste $X_2$, $X_3$, $X_4$ oder $X_5$ von Wasserstoff verschieden ist, oder mindetsnes $X_2$ und $X_3$ zusammen oder $X_4$ und $X_5$ zusammen einen zweiwertigen, durch zwei Wasserstoffatome ersetzbaren Rest darstellen, und R und m obige Bedeutungen haben, oder in einem Salz davon, das von Wasserstoff verschiedene $X_2$, $X_3$, $X_4$ oder $X_5$ bzw. $X_2$ und $X_3$ zusammen oder $X_4$ und $X_5$ zusammen durch Wasserstoff ersetzt, oder

c) in einer Verbindung der Formel

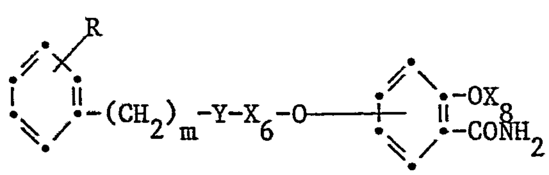 (V),

worin $X_6$ eine reduzierte Gruppe der Formeln

$-CH=N-CH_2-CH_2-$ (Va), bzw. $-CH_2-N=CH-CH_2-$ (Vb), oder

$-C(=X_7)-N(X_8)-CH_2-CH_2-$ (IVc) bzw. $-CH_2-N(X_8)-C(=X_7)-CH_2-$ (Vd),

oder eine Gruppe $-CH_2-N(X_8)-CH_2-CH_2-$ (Ve), ist, worin $X_7$ den Oxo- oder Thioxorest und $X_8$ Wasserstoff oder einen unter den Bedingungen für die Reduktion von $X_6$ und/oder Y durch Wasserstoff ersetzbaren Rest darstellt und Y für einen Rest der Formel -CO- (Vf) oder $-CH(OX_8)-$ (Vg) steht, in der $X_8$ die vorstehend angegebene Bedeutung hat, R und m obige Bedeutung haben, wobei stets $X_6$ eine reduzierbare Gruppe Va bis Vd und/oder Y eine Carbonylgruppe Vf ist, diese Gruppe(n) reduziert und im gleichen Arbeitsgang die von Wasserstoff verschiedenen Gruppen $X_8$ durch Wasserstoff ersetzt, oder

d) eine Verbindung der Formel

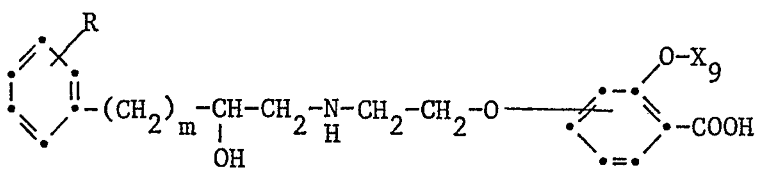

(VI),

worin $X_9$ Wasserstoff oder eine mittels Ammonolyse abspaltbare Gruppe bedeutet, R und m obige Bedeutung haben, oder ein reaktionsfähiges Derivat einer der in Formel VI definierten Carbonsäuren, mit Ammoniak umsetzt und gegebenenfalls vorhandene Reste $X_9$ abspaltet und durch Wasserstoff ersetzt, oder

e) in einer Verbindung der Formel

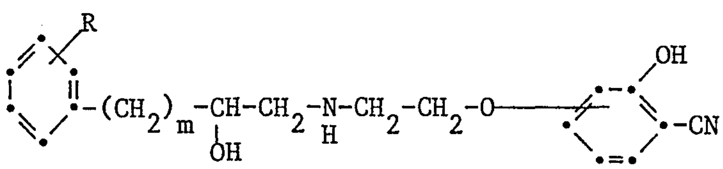

(VII),

worin eine oder beide Hydroxygruppen gegebenenfalls durch solche mittels Hydrolyse abspaltbaren und durch Wasserstoff ersetzbaren Gruppen geschützt sind, die unter den Bedingungen des Verfahrens abgespalten und durch Wasserstoff ersetzt werden, die Gruppe -CN mittels Hydrolyse in die Gruppe $-CONH_2$ umwandelt, oder

f) in einer Verbindung der Formel

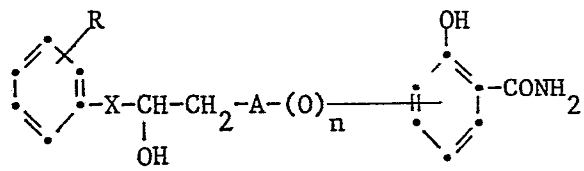

(VIII),

worin A für die Gruppe $-\underset{H}{N}-CH_2-CH_2-$ oder $-N=CH-CH_2$ steht, X eine mittels Reduktion, einschliesslich Hydrogenolyse, in eine 1,3-Propylengruppe überführbare Gruppe darstellt, und eine oder beide Hydroxygruppen durch solche mittels Reduktion, einschliesslich Hydrogenolyse, abspaltbaren und durch Wasserstoff ersetzbaren Gruppen geschützt sind, die unter den Bedingungen des Verfahrens abgespalten und durch Wasserstoff ersetzt werden, die Gruppe X mittels Reduktion einschliesslich Hydrogenolyse in eine 1,3-Propylengruppe und gleichzeitig eine gegebenenfalls vorhandene ungesättigte Gruppe A in die gesättigte Gruppe A umwandelt und, wenn erwünscht, eine erhaltene freie Verbindung in ein Salz oder ein erhaltenes Salz in eine freie Verbindung überführt, und/oder, wenn erwünscht, ein erhaltenes Racemat in die optischen Antipoden auftrennt.

23. Die nach dem Verfahren des Anspruchs 22 erhältlichen Verbindungen.

Europäisches Patentamt

# EUROPÄISCHER RECHERCHENBERICHT

0069838

Nummer der Anmeldung

EP 82 10 3892

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | EP-A-0 005 848 (CIBA-GEIGY)<br>*Patentansprüche*<br>--- | 1,11-22 | C 07 C 103/26<br>A 61 K 31/165 |
| P,X | EP-A-0 030 030 (CIBA-GEIGY)<br>*Patentansprüche*<br>----- | 1,11-22 | |
| | | | **RECHERCHIERTE SACHGEBIETE (Int. Cl. 3)** |
| | | | C 07 C 103/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 16-08-1982 | MOREAU J.M. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82